# EUROPEAN PATENT SPECIFICATION

(11) **EP 2 851 432 B1**
(45) Date of publication and mention of the grant of the patent: **09.01.2019**
(21) Application number: 14177669.0
(22) Date of filing: 03.11.2008
(51) Int. Cl.: C12Q 1/6883

(54) **RCA locus analysis to assess susceptibility to AMD**
RCA-Locus-Analyse zur Beurteilung der Anfälligkeit für AMD
Analyse de locus de RCA pour évaluer la sensibilité à l'AMD

(30) Priority: 01.11.2007 US 984702 P
(43) Date of publication of application: 25.03.2015
(62) Divisional of application: 08843989.8
(73) Proprietor: University of Iowa Research Foundation, Iowa City, IA 52242-5500 (US)
(72) Inventor: Hageman, Gregory S, Salt Lake City, UT 84132 (US)
(74) Representative: Avidity IP

(56) References cited:
- WO-A2-2006/062716
- US-A1- 2007 020 647
- US-A1- 2007 037 183
- ZHANG HONG ET AL: "The NEI/NCBI dbGAP database: genotypes and haplotypes that may specifically predispose to risk of neovascular age-related macular degeneration.", BMC MEDICAL GENETICS 2008 LNKD- PUBMED:18541031, vol. 9, 2008, page 51, XP021034355, ISSN: 1471-2350
- HAGEMAN GREGORY S ET AL: "Extended haplotypes in the complement factor H (CFH) and CFH-related (CFHR) family of genes protect against age-related macular degeneration: characterization, ethnic distribution and evolutionary implications", ANNALS OF MEDICINE, TAYLOR & FRANCIS A B, SE LNKD- DOI:10.1080/07853890601097030, vol. 38, no. 8, 2006, pages 592-604, XP002457295, ISSN: 0785-3890
- GEHRS K M ET AL: "Age-related macular degeneration - Emerging pathogenetic and therapeutic concepts", ANNALS OF MEDICINE, TAYLOR & FRANCIS A B, SE LNKD- DOI:10.1080/07853890600946724, vol. 38, no. 7, 2006, pages 450-471, XP009105015, ISSN: 0785-3890
- GREGORY S HAGEMAN ET AL: "Clinical validation of a genetic model to estimate the risk of developing choroidal neovascular age-related macular degeneration", HUMAN GENOMICS, BIOMED CENTRAL LTD, LONDON, UK, vol. 5, no. 5, July 2011 (2011-07), pages 420-440, XP021127006, ISSN: 1479-7364, DOI: 10.1186/1479-7364-5-5-420

## Description

### FIELD OF THE INVENTION

The disclosure relates to risk determination, diagnosis and prognosis of complement-related disorders such as age-related macular degeneration (AMD) and membranoproliferative glomerulonephritis type 2 (MPGNII).

### BACKGROUND OF THE INVENTION

Age-related macular degeneration (AMD) is the leading cause of irreversible vision loss in the developed world, affecting approximately 15% of individuals over the age of 60. The prevalence of AMD increases with age: mild, or early, forms occur in nearly 30%, and advanced forms in about 7%, of the population that is 75 years and older. Clinically, AMD is characterized by a progressive loss of central vision attributable to degenerative changes that occur in the macula, a specialized region of the neural retina and underlying tissues. In the most severe, or exudative, form of the disease neovascular fronds derived from the choroidal vasculature breach Bruch's membrane and the retinal pigment epithelium (RPE) typically leading to detachment and subsequent degeneration of the retina.

Numerous studies have implicated inflammation in the pathobiology of AMD (Anderson et al. (2002) Am. J. Ophthalmol. 134:41 1-31; Hageman et al. (2001) Prog. Retin. Eye Res. 20:705-32; Mullins et al. (2000) Faseb_J. 14:835-46; Johnson et al. (2001) Exp. Eye Res._73:887-96; Crabb et al. (2002) PNAS 99:14682-7; Bok (2005) PNAS 102:7053-4).

Dysfunction of the complement pathway may induce significant bystander damage to macular cells, leading to atrophy, degeneration, and the elaboration of choroidal neovascular membranes, similar to damage that occurs in other complement-mediated disease processes (Hageman et al. (2005) PNAS 102:7227-32: Morgan and Walport (1991) Immunol. Today 12:301- 6; Kinoshita (1991) Immunol. Today 12:291-5; Holers and Thurman (2004) Mol. Immunol. 41: 147-52).

AMD, a late-onset complex disorder, appears to be caused and/or modulated by a combination of genetic and environmental factors. According to the prevailing hypothesis, the majority of AMD cases is not a collection of multiple single-gene disorders, but instead represents a quantitative phenotype, an expression of interaction of multiple susceptibility loci. The number of loci involved, the attributable risk conferred, and the interactions between various loci remain obscure, but significant progress has been made in determining the genetic contribution to these diseases. *See,* for example, U.S. Patent Publication No. 20070020647, U.S. Patent Publication No. 20060281120, PCT publication WO 2008/013893, and U.S. Patent Publication No. 20080152659.

Thus, variations in complement-related genes have been found to be correlated with AMD. A common haplotype in the complement regulatory gene factor H (*HF1*/*CFH*) predisposes individuals to age-related macular degeneration. Hageman et al., 2005, Proc. Nat'l Acad Sci 102: 7227-32. Similarly, the non-synonymous polymorphism at amino acid position 1210 in exon 22 of the Factor H gene is strongly associated with AMD. *See, e.g.,* Hageman et al. WO 2006/088950; Hageman et al. WO2007/095287 and Hughes et al., 2006, Nat Genet. 38:458-62. Deletions and other variations in other genes of the RCA locus (such as CFH-related 3 [FHR3] and CFH-related 1 [FHR1], among others) have also been correlated with AMD. See, for example, International Publication No. WO2008/008986, and Hughes et al., 2006, Nat Genet. 38:458-62.

Membranoproliferative glomerulonephritis type 2 (MPGNII), which is also known as dense deposit disease, is a rare disease that is associated with uncontrolled systemic activation of the alternative pathway of the complement cascade. The disease is characterized by the deposition of abnormal electron-dense material comprised of C3 and C3c within the renal glomerular basement membrane, which eventually leads to renal failure. Interestingly, many patients with MPGNII develop macular drusen, RPE detachments and choroidal neovascular membranes that are clinically and compositionally indistinguishable from those that form in AMD, although they are often detected in the second decade of life (Mullins et al., 2001, Eye 15, 290-395). Thus, MPGNII may represent an early form of AMD.

Analysis of single polynucleotide polymorphisms (SNPs) is a powerful technique for diagnosis and/or determination of risk for complement-related disorders such as AMD and MPGNII.

### SUMMARY

The invention arises, in part, from a high density, large sample size, genetic association study designed to detect genetic characteristics associated with complement cascade dysregulation diseases such as AMD and MPGNII. The study revealed a large number of new SNPs never before reported and a still larger number of SNPs (and/or combination of certain SNPs) which were not previously reported to be associated with risk for, or protection from, these diseases. The invention disclosed herein thus relates to the discovery of polymorphisms within the Regulation of Complement Activation (RCA) locus that are associated with the development of age-related macular degeneration (AMD) and membranoproliferative glomerulonephritis type 2 (MPGNII). The invention provides methods of screening for individuals at risk of developing these diseases and/or for predicting the likely progression of early- or mid-stage established disease and/or for predicting the likely outcome of a particular therapeutic or prophylactic strategy.

The invention provides an *in vitro* method of determining an individual's risk for age-related macular degeneration (AMD) comprising screening for the presence or absence of a genetic profile, wherein the genetic profile comprises the individual being homozygous for the G allele at the polymorphic site rs1409153, and wherein the presence of said genetic profile is indicative of increased risk.

### DETAILED DESCRIPTION OF THE INVENTION

Disclosed is a diagnostic method of determining an individual's propensity to complement dysregulation comprising screening (directly or indirectly) for the presence or absence of a genetic profile characterized by polymorphisms in the individual's genome associated with complement dysregulation, wherein the presence of said genetic profile is indicative of the individual's risk of complement dysregulation. The profile may reveal that the individual's risk is increased, or decreased, as the profile may evidence increased risk for, or increased protection from, developing AMD and/or MPGNII. A genetic profile associated with complement dysregulation comprises one or more, typically multiple, single nucleotide polymorphisms selected from Table I, Table IA, and/or Table II. In certain embodiments, a genetic profile associated with complement dysregulation comprises any combination of at least 2, at least 5, or at least 10 single nucleotide polymorphisms selected from Table I, Table IA, and/or Table II.

Further disclosed is a diagnostic method of determining an individual's propensity to develop, or for predicting the course of progression, of AMD, comprising screening (directly or indirectly) for the presence or absence of a genetic profile in the regulation of complement activation (RCA) locus of human chromosome 1 extending from complement factor H related 1 (FHR1) through complement factor 13B (F13B), which are informative of an individual's (increased or decreased) risk for developing AMD. A genetic profile in the RCA locus comprises one or more, typically multiple, single nucleotide polymorphisms selected from Table I and/or Table IA. In other embodiments, a genetic profile in the RCA locus comprises any combination of at least 2, at least 5, or at least 10 single nucleotide polymorphisms selected from Table I and/or Table IA.

The disclosed embodiment of a method for determining an individual's propensity to develop, or for predicting the course of progression, of age-related macular degeneration, comprises screening for a combination of at least one, typically multiple, risk-associated polymorphism and at least one, typically multiple, protective polymorphism set forth in Table I, Table IA, and/or Table II. For example, the method may comprise screening for at least rs1409153, rs10922153, rs12066959, and rs12027476. Risk polymorphisms indicate that an individual has increased susceptibility to developing AMD and/or MPGNII relative to the control population. Protective polymorphisms indicate that the individual has a reduced likelihood of developing AMD and/or MPGNII relative to the control population. Neutral polymorphisms do not segregate significantly with risk or protection, and have limited or no diagnostic or prognostic value. Additional, previously known informative polymorphisms may and typically will be included in the screen. For example, additional risk-associated polymorphisms may include rs1061170, rs203674, rs1061147, rs2274700, rs12097550, rs203674, a polymorphism in exon 22 of CFH (R1210C), rs9427661, rs9427662, rs10490924, rs11200638, rs2230199, rs2511989, rs3753395, rs1410996, rs393955, rs403846, rs1329421, rs10801554, rs12144939, rs12124794, rs2284664, rs16840422, rs6695321, and rs2511989. Additional protection-associated polymorphisms may include: rs800292, rs3766404, rs529825, rs641153, rs4151667, rs547154, and rs9332739.

The disclosed embodiment of a method for determining an individual's propensity to develop or for predicting the course of progression of AMD or MPGNII, comprises screening additionally for deletions within the RCA locus that are associated with AMD or MPGNII risk or protection. An exemplary deletion that is protective of AMD is a deletion at least portions of the FHR3 and FHR1 genes. *See, e.g.,* Hageman et al., 2006, "Extended haplotypes in the complement factor H (CFH) and CFH-related (CFHR) family of genes protect against age-related macular degeneration: characterization, ethnic distribution and evolutionary implications ," Ann Med. 38:592-604 and US Patent Publication No. 2008/152659.

Further disclosed is a diagnostic method of determining an individual's propensity to develop or for predicting the course of progression of membranoproliferative glomerulonephritis type 2 (MPGNII), comprising screening for the presence or absence of a genetic profile in the regulation of complement activation (RCA) locus of chromosome 1 extending from complement factor H (CFH) through complement factor 13B (F13B). In one embodiment, a genetic profile in the RCA locus comprises one or more, typically multiple, single nucleotide polymorphisms selected from Table II. In other embodiments, a genetic profile in the RCA locus comprises at least 2, at least 5, or at least 10 single nucleotide polymorphisms selected from Table II, and of course may include additional polymorphisms known to be associated with MPGN-II risk or protection.

A disclosed embodiment relates to inspecting a data set indicative of genetic characteristics previously derived from analysis of the individual's genome. A data set of genetic characteristics of the individual may include, for example, a listing of single nucleotide polymorphisms in the patient's genome or a a complete or partial sequence of the individual's genomic DNA. The methods include obtaining and analyzing a nucleic acid sample (*e.g*., DNA or RNA) from an individual to determine whether the DNA contains informative polymorphisms in the RCA locus. In another embodiment, the methods include obtaining a biological sample from the individual and analyzing the sample from the individual to determine whether the individual's proteome contains an allelic variant isoform that is a consequence of the presence of a polymorphisms in the individual's genome.

A disclosed embodiment relates to treating, preventing, or delaying development of symptoms of AMD and/or MPGNII in an individual (*e.g*., an individual in whom a genetic profile indicative of elevated risk of developing AMD and/or MPGNII is detected), comprising prophylactically or therapeutically treating an individual identified as having a genetic profile including one or more single nucleotide polymorphisms selected from Table I, Table IA, or Table II.

The disclosed embodiment of treating or preventing AMD and/or MPGNII in an individual comprises prophylatically or therapeutically treating the individual by administering a composition comprising a Factor H polypeptide. The Factor H polypeptide may be a wild type Factor H polypeptide or a variant Factor H polypeptide. The Factor H polypeptide may be a Factor H polypeptide with a sequence encoded by a protective or neutral allele. In one embodiment, the Factor H polypeptide is encoded by a Factor H protective haplotype. A protective Factor H haplotype can encode an isoleucine residue at amino acid position 62 and/or an amino acid other than a histidine at amino acid position 402. For example, a Factor H polypeptide can comprise an isoleucine residue at amino acid position 62, a tyrosine residue at amino acid position 402, and/or an arginine residue at amino acid position 1210. Exemplary Factor H protective haplotypes include the H2 haplotype or the H4 haplotype. Alternatively, the Factor H polypeptide may be encoded by a Factor H neutral haplotype. A neutral haplotype encodes an amino acid other than an isoleucine at amino acid position 62 and an amino acid other than a histidine at amino acid position 402. Exemplary Factor H neutral haplotypes include the H3 haplotype or the H5 haplotype. For details on therapeutic forms of CFH, and how to make and use them, *see* U.S. Patent Publication No. 20070060247.

The disclosed embodiment of treating or preventing AMD in an individual includes prophylactically or therapeutically treating the individual by inhibiting Factor B and/or C2 in the individual. Factor B can be inhibited, for example, by administering an antibody or other protein (*e.g.,* an antibody variable domain, an addressable fibronectin protein, *etc*.) that binds Factor B. Alternatively, Factor B can be inhibited by administering a nucleic acid inhibiting Factor B expression or activity, such as an inhibitory RNA, a nucleic acid encoding an inhibitory RNA, an antisense nucleic acid, or an aptamer, or by administering a small molecule that interferes with Factor B activity (*e.g*., an inhibitor of the protease activity of Factor B). C2 can be inhibited, for example, by administering an antibody or other protein (*e.g.,* an antibody variable domain, an addressable fibronectin protein, *etc.*) that binds C2. Alternatively, C2 can be inhibited by administering a nucleic acid inhibiting C2 expression or activity, such as an inhibitory RNA, a nucleic acid encoding an inhibitory RNA, an antisense nucleic acid, or an aptamer, or by administering a small molecule that interferes with C2 activity (e.g., an inhibitor of the protease activity of C2).

The disclosed embodiment of treating or preventing AMD in an individual includes prophylactically or therapeutically treating the individual by inhibiting HTRA1 in the individual. HTRA1 can be inhibited, for example, by administering an antibody or other protein (*e.g.* an antibody variable domain, an addressable fibronectin protein, *etc*.) that binds HTRA1. Alternatively, HTRA1 can be inhibited by administering a nucleic acid inhibiting HTRA1 expression or activity, such as an inhibitory RNA, a nucleic acid encoding an inhibitory RNA, an antisense nucleic acid, or an aptamer, or by administering a small molecule that interferes with HTRA1 activity (e.g. an inhibitor of the protease activity of HTRA1).

Further disclosed are detectably labeled oligonucleotide probes or primers for hybridization with DNA sequence in the vicinity of at least one polymorphism to facilitate identification of the base present in the individual's genome. In one embodiment, a set of oligonucleotide primers hybridizes to a region of the RCA locus adjacent to at least one polymorphism for inducing amplification thereof, thereby facilitating sequencing of the region and determination of the base present in the individual's genome at the sites of the polymorphism. Preferred polymorphisms for detection include the polymorphisms listed in Tables I, IA, and II. Further, one of skill in the art will appreciate that other methods for detecting polymorphisms are well known in the art.

Further disclosed is a healthcare method that includes authorizing the administration of, or authorizing payment for the administration of, a diagnostic assay to determine an individual's susceptibility for development or progression of AMD and/or MPGNII comprising screening for the presence or absence of a genetic profile in the RCA locus of chromosome one extending from CFH to F13B, wherein the genetic profile comprises one or more SNPs selected from Table I, Table IA and/or Table II.

### BRIEF DESCRIPTION OF DRAWINGS

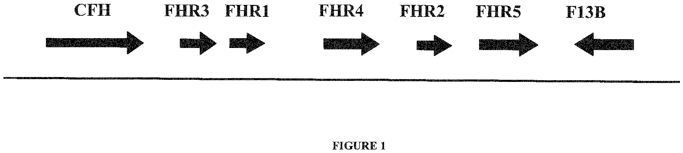

Figure 1 is a schematic diagram depicting the order of some genes within the regulation of complement activation (RCA) locus.

### FURTHER DETAILED DESCRIPTION OF THE INVENTION

### I. Definitions and Conventions

The term "polymorphism" refers to the occurrence of two or more genetically determined alternative sequences or alleles in a population. Each divergent sequence is termed an allele, and can be part of a gene or located within an intergenic or non-genic sequence. A diallelic polymorphism has two alleles, and a triallelic polymorphism has three alleles. Diploid organisms can contain two alleles and may be homozygous or heterozygous for allelic forms. The first identified allelic form is arbitrarily designated the reference form or allele; other allelic forms are designated as alternative or variant alleles. The most frequently occurring allelic form in a selected population is typically referred to as the wild-type form.

A "polymorphic site" is the position or locus at which sequence divergence occurs at the nucleic acid level and is sometimes reflected at the amino acid level. The polymorphic region or polymorphic site refers to a region of the nucleic acid where the nucleotide difference that distinguishes the variants occurs, or, for amino acid sequences, a region of the amino acid sequence where the amino acid difference that distinguishes the protein variants occurs. A polymorphic site can be as small as one base pair, often termed a "single nucleotide polymorphism" (SNP). The SNPs can be any SNPs in loci identified herein, including intragenic SNPs in exons, introns, or upstream or downstream regions of a gene, as well as SNPs that are located outside gene sequences. Examples of such SNPs include, but are not limited to, those provided in the tables hereinbelow.

Individual amino acids in a sequence are represented herein as AN or NA, wherein A is the amino acid in the sequence and N is the position in the sequence. In the case that position N is polymorphic, it is convenient to designate the more frequent variant as A₁N and the less frequent variant as NA₂. Alternatively, the polymorphic site, N, is represented as A₁NA₂, wherein A₁ is the amino acid in the more common variant and A₂ is the amino acid in the less common variant. Either the one-letter or three-letter codes are used for designating amino acids (*see* Lehninger, Biochemistry 2nd ed., 1975, Worth Publishers, Inc. New York, N.Y.: pages 73 -75). For example, I50V represents a single-amino-acid polymorphism at amino acid position 50 of a given protein, wherein isoleucine is present in the more frequent protein variant in the population and valine is present in the less frequent variant.

Similar nomenclature may be used in reference to nucleic acid sequences. In the Tables provided herein, each SNP is depicted by "N₁/N₂" where N₁ is a nucleotide present in a first allele referred to as Allele 1, and N₂ is another nucleotide present in a second allele referred to as Allele 2. It will be clear to those of skill in the art that in a double-stranded form, the complementary strand of each allele will contain the complementary base at the polymorphic position.

The term "genotype" as used herein denotes one or more polymorphisms of interest found in an individual, for example, within a gene of interest. Diploid individuals have a genotype that comprises two different sequences (heterozygous) or one sequence (homozygous) at a polymorphic site.

The term "haplotype" refers to a DNA sequence comprising one or more polymorphisms of interest contained on a subregion of a single chromosome of an individual. A haplotype can refer to a set of polymorphisms in a single gene, an intergenic sequence, or in larger sequences including both gene and intergenic sequences, e.g., a collection of genes, or of genes and intergenic sequences. For example, a haplotype can refer to a set of polymorphisms in the regulation of complement activation (RCA) locus, which includes gene sequences for complement factor H (CFH), FHR3, FHR1, FHR4, FHR2, FHR5, and F13B and intergenic sequences (*i.e.,* intervening intergenic sequences, upstream sequences, and downstream sequences that are in linkage disequilibrium with polymorphisms in the genic region). The term "haplotype" can refer to a set of single nucleotide polymorphisms (SNPs) found to be statistically associated on a single chromosome. A haplotype can also refer to a combination of polymorphisms (*e.g.,* SNPs) and other genetic markers (*e.g.,* a deletion) found to be statistically associated on a single chromosome. A haplotype, for instance, can also be a set of maternally inherited alleles, or a set of paternally inherited alleles, at any locus.

The term "genetic profile," as used herein, refers to a collection of one or more single nucleotide polymorphisms comprising polymorphisms shown in Table I (AMD) or Table II (MPGNII), optionally in combination with other genetic characteristics such as deletions, additions or duplications, and optionally combined with other SNPs known to be associated with AMD (or MPGNII) risk or protection. Thus, a genetic profile, as the phrase is used herein, is not limited to a set of characteristics defining a haplotype, and may comprise SNPs from diverse regions of the genome. For example, a genetic profile for AMD comprises one or a subset of single nucleotide polymorphisms selected from Table I and/or Table IA, optionally in combination with other genetic characteristics known to be associated with AMD. It is understood that while one SNP in a genetic profile may be informative of an individual's increased or decreased risk (*i.e.,* an individual's propensity or susceptibility) to develop a complement-related disease such as AMD and/or MPGNII, more than one SNP in a genetic profile may and typically will be analyzed and will be more informative of an individual's increased or decreased risk of developing a complement-related disease. A genetic profile may include at least one SNP disclosed herein in combination with other polymorphisms or genetic markers (*e.g.,* a deletion) and/or environmental factors (*e.g.,* smoking or obesity) known to be associated with AMD and/or MPGNII. In some cases, a SNP may reflect a change in regulatory or protein coding sequences that change gene product levels or activity in a manner that results in increased likelihood of development of a disease. In addition, it will be understood by a person of skill in the art that one or more SNPs that are part of a genetic profile may be in linkage disequilibrium with, and serve as a proxy or surrogate marker for another genetic marker or polymorphism that is causative, protective, or otherwise informative of disease.

The term "gene," as used herein, refers to a region of a DNA sequence that encodes a polypeptide or protein, intronic sequences, promoter regions, and upstream (*i.e.,* proximal) and downstream (*i.e.,* distal) non-coding transcription control regions (*e.g.,* enhancer and/or repressor regions).

The term "allele," as used herein, refers to a sequence variant of a genetic sequence (*e.g.,* typically a gene sequence as described hereinabove, optionally a protein coding sequence). For purposes of this application, alleles can but need not be located within a gene sequence. Alleles can be identified with respect to one or more polymorphic positions such as SNPs, while the rest of the gene sequence can remain unspecified. For example, an allele may be defined by the nucleotide present at a single SNP, or by the nucleotides present at a plurality of SNPs. In certain embodiments of the invention, an allele is defined by the genotypes of at least 1, 2, 4, 8 or 16 or more SNPs (including those provided in Tables I, IA, and II below) in a gene.

A "causative" SNP is a SNP having an allele that is directly responsible for a difference in risk of development or progression of AMD. Generally, a causative SNP has an allele producing an alteration in gene expression or in the expression, structure, and/or function of a gene product, and therefore is most predictive of a possible clinical phenotype. One such class includes SNPs falling within regions of genes encoding a polypeptide product, *i.e.* "coding SNPs" (cSNPs). These SNPs may result in an alteration of the amino acid sequence of the polypeptide product (*i.e.,* non-synonymous codon changes) and give rise to the expression of a defective or other variant protein. Furthermore, in the case of nonsense mutations, a SNP may lead to premature termination of a polypeptide product. Such variant products can result in a pathological condition, e.g., genetic disease. Examples of genes in which a SNP within a coding sequence causes a genetic disease include sickle cell anemia and cystic fibrosis.

Causative SNPs do not necessarily have to occur in coding regions; causative SNPs can occur in, for example, any genetic region that can ultimately affect the expression, structure, and/or activity of the protein encoded by a nucleic acid. Such genetic regions include, for example, those involved in transcription, such as SNPs in transcription factor binding domains, SNPs in promoter regions, in areas involved in transcript processing, such as SNPs at intron-exon boundaries that may cause defective splicing, or SNPs in mRNA processing signal sequences such as polyadenylation signal regions. Some SNPs that are not causative SNPs nevertheless are in close association with, and therefore segregate with, a disease-causing sequence. In this situation, the presence of a SNP correlates with the presence of, or predisposition to, or an increased risk in developing the disease. These SNPs, although not causative, are nonetheless also useful for diagnostics, disease predisposition screening, and other uses.

An "informative" or "risk-informative" SNP refers to any SNP whose sequence in an individual provides information about that individual's relative risk of development or progression of AMD. An informative SNP need not be causative. Indeed, many informative SNPs have no apparent effect on any gene product, but are in linkage disequilibrium with a causative SNP. In such cases, as a general matter, the SNP is increasingly informative when it is more tightly in linkage disequilibrium with a causative SNP. For various informative SNPs, the relative risk of development or progression of AMD is indicated by the presence or absence of a particular allele and/or by the presence or absence of a particular diploid genotype.

The term "linkage" refers to the tendency of genes, alleles, loci, or genetic markers to be inherited together as a result of their location on the same chromosome or as a result of other factors. Linkage can be measured by percent recombination between the two genes, alleles, loci, or genetic markers. Some linked markers may be present within the same gene or gene cluster.

In population genetics, linkage disequilibrium is the non-random association of alleles at two or more loci, not necessarily on the same chromosome. It is not the same as linkage, which describes the association of two or more loci on a chromosome with limited recombination between them. Linkage disequilibrium describes a situation in which some combinations of alleles or genetic markers occur more or less frequently in a population than would be expected from a random formation of haplotypes from alleles based on their frequencies. Non-random associations between polymorphisms at different loci are measured by the degree of linkage disequilibrium (LD). The level of linkage disequilibrium is influenced by a number of factors including genetic linkage, the rate of recombination, the rate of mutation, random drift, non-random mating, and population structure. Linkage disequilibrium" or "allelic association" thus means the non-random association of a particular allele or genetic marker with another specific allele or genetic marker more frequently than expected by chance for any particular allele frequency in the population. A marker in linkage disequilibrium with an informative marker, such as one of the SNPs listed in Tables I, IA, or II can be useful in detecting susceptibility to disease. A SNP that is in linkage disequilibrium with a causative, protective, or otherwise informative SNP or genetic marker is referred to as a "proxy" or "surrogate" SNP. A proxy SNP may be in at least 50%, 60%, or 70% in linkage disequilibrium with the causative SNP, and preferably is at least about 80%, 90%, and most preferably 95%, or about 100% in LD with the genetic marker.

A "nucleic acid," "polynucleotide," or "oligonucleotide" is a polymeric form of nucleotides of any length, may be DNA or RNA, and may be single- or double-stranded. The polymer may include, without limitation, natural nucleosides (*i.e*., adenosine, thymidine, guanosine, cytidine, uridine, deoxyadenosine, deoxythymidine, deoxyguanosine, and deoxycytidine), nucleoside analogs (e.g., 2-aminoadenosine, 2-thiothymidine, inosine, pyrrolo-pyrimidine, 3-methyl adenosine, 5-methylcytidine, C5-bromouridine, C5-fluorouridine, C5-iodouridine, C5-propynyl-uridine, C5-propynyl-cytidine, C5-methylcytidine, 7-deazaadenosine, 7-deazaguanosine, 8-oxoadenosine, 8-oxoguanosine, O(6)-methylguanine, and 2-thiocytidine), chemically modified bases, biologically modified bases (*e.g.,* methylated bases), intercalated bases, modified sugars (*e.g.,* 2'-fluororibose, ribose, 2'-deoxyribose, arabinose, and hexose), or modified phosphate groups (e.g., phosphorothioates and 5'-N-phosphoramidite linkages). Nucleic acids and oligonucleotides may also include other polymers of bases having a modified backbone, such as a locked nucleic acid (LNA), a peptide nucleic acid (PNA), a threose nucleic acid (TNA) and any other polymers capable of serving as a template for an amplification reaction using an amplification technique, for example, a polymerase chain reaction, a ligase chain reaction, or non-enzymatic template-directed replication.

Oligonucleotides are usually prepared by synthetic means. Nucleic acids include segments of DNA, or their complements spanning any one of the polymorphic sites shown in the Tables provided herein. Except where otherwise clear from context, reference to one strand of a nucleic acid also refers to its complement strand. The segments are usually between 5 and 100 contiguous bases, and often range from a lower limit of 5, 10, 12, 15, 20, or 25 nucleotides to an upper limit of 10, 15, 20, 25, 30, 50 or 100 nucleotides (where the upper limit is greater than the lower limit). Nucleic acids between 5-10, 5-20, 10-20, 12-30, 15-30, 10-50, 20-50 or 20-100 bases are common. The polymorphic site can occur within any position of the segment. The segments can be from any of the allelic forms of DNA shown in the Tables provided herein.

"Hybridization probes" are nucleic acids capable of binding in a base-specific manner to a complementary strand of nucleic acid. Such probes include nucleic acids and peptide nucleic acids. Hybridization is usually performed under stringent conditions which are known in the art. A hybridization probe may include a "primer."

The term "primer" refers to a single-stranded oligonucleotide capable of acting as a point of initiation of template-directed DNA synthesis under appropriate conditions, in an appropriate buffer and at a suitable temperature. The appropriate length of a primer depends on the intended use of the primer, but typically ranges from 15 to 30 nucleotides. A primer sequence need not be exactly complementary to a template, but must be sufficiently complementary to hybridize with a template. The term "primer site" refers to the area of the target DNA to which a primer hybridizes. The term "primer pair" means a set of primers including a 5' upstream primer, which hybridizes to the 5' end of the DNA sequence to be amplified and a 3' downstream primer, which hybridizes to the complement of the 3' end of the sequence to be amplified.

The nucleic acids, including any primers, probes and/or oligonucleotides can be synthesized using a variety of techniques currently available, such as by chemical or biochemical synthesis, and by *in vitro* or *in vivo* expression from recombinant nucleic acid molecules, e.g., bacterial or retroviral vectors. For example, DNA can be synthesized using conventional nucleotide phosphoramidite chemistry and the instruments available from Applied Biosystems, Inc. (Foster City, Calif.); DuPont (Wilmington, Del.); or Milligen (Bedford, Mass.). When desired, the nucleic acids can be labeled using methodologies well known in the art such as described in U.S. Pat. Nos. 5,464,746; 5,424,414; and 4,948,882. In addition, the nucleic acids can comprise uncommon and/or modified nucleotide residues or non-nucleotide residues, such as those known in the art.

An "isolated" nucleic acid molecule, as used herein, is one that is separated from nucleotide sequences which flank the nucleic acid molecule in nature and/or has been completely or partially purified from other biological material (e.g., protein) normally associated with the nucleic acid. For instance, recombinant DNA molecules in heterologous organisms, as well as partially or substantially purified DNA molecules in solution, are "isolated" for present purposes.

The term "target region" refers to a region of a nucleic acid which is to be analyzed and usually includes at least one polymorphic site.

"Stringent" as used herein refers to hybridization and wash conditions at 50° C or higher. Other stringent hybridization conditions may also be selected. Generally, stringent conditions are selected to be about 5° C lower than the thermal melting point (Tₘ) for the specific sequence at a defined ionic strength and pH. The Tₘ is the temperature (under defined ionic strength and pH) at which 50% of the target sequence hybridizes to a perfectly matched probe. Typically, stringent conditions will be those in which the salt concentration is at least about 0.02 molar at pH 7 and the temperature is at least about 50° C. As other factors may significantly affect the stringency of hybridization, including, among others, base composition, length of the nucleic acid strands, the presence of organic solvents, the extent of base mismatching, and the combination of parameters is more important than the absolute measure of any one.

Generally, increased or decreased risk-associated with a polymorphism or genetic profile for a disease is indicated by an increased or decreased frequency, respectively, of the disease in a population or individuals harboring the polymorphism or genetic profile, as compared to otherwise similar individuals, who are for instance matched by age, by population, and/or by presence or absence of other polymorphisms associated with risk for the same or similar diseases. The risk effect of a polymorphism can be of different magnitude in different populations. A polymorphism, haplotype, or genetic profile can be negatively associated ("protective polymorphism") or positively associated ("predisposing polymorphism") with a complement-related disease such as AMD and MPGNII. The presence of a predisposing genetic profile in an individual can indicate that the individual has an increased risk for the disease relative to an individual with a different profile. Conversely, the presence of a protective polymorphism or genetic profile in an individual can indicate that the individual has a decreased risk for the disease relative to an individual without the polymorphism or profile.

The terms "susceptibility," "propensity," and "risk" refer to either an increased or decreased likelihood of an individual developing a disorder (*e.g.,* a condition, illness, disorder or disease) relative to a control and/or non-diseased population. In one example, the control population may be individuals in the population (e.g., matched by age, gender, race and/or ethnicity) without the disorder, or without the genotype or phenotype assayed for.

The terms "diagnose" and "diagnosis" refer to the ability to determine or identify whether an individual has a particular disorder (*e.g.,* a condition, illness, disorder or disease). The term prognose or prognosis refers to the ability to predict the course of the disease and/or to predict the likely outcome of a particular therapeutic or prophylactic strategy.

The term "screen" or "screening" as used herein has a broad meaning. It includes processes intended for the diagnosis or for determining the susceptibility, propensity, risk, or risk assessment of an asymptomatic subject for developing a disorder later in life. Screening also includes the prognosis of a subject, *i.e.,* when a subject has been diagnosed with a disorder, determining in advance the progress of the disorder as well as the assessment of efficacy of therapy options to treat a disorder. Screening can be done by examining a presenting individual's DNA, RNA, or in some cases, protein, to assess the presence or absence of the various SNPs disclosed herein (and typically other SNPs and genetic or behavioral characteristics) so as to determine where the individual lies on the spectrum of disease risk-neutrality-protection. Proxy SNPs may substitute for any of these SNPs. A sample such as a blood sample may be taken from the individual for purposes of conducting the genetic testing using methods known in the art or yet to be developed. Alternatively, if a health provider has access to a pre-produced data set recording all or part of the individual's genome (*e.g.,* a listing of SNPs in the patient;s genome) screening may be done simply by inspection of the database, optimally by computerized inspection. Screening may further comprise the step of producing a report identifying the individual and the identity of alleles at the site of at least one or more polymorphisms shown in Table I, Table IA or Table II.

The term "regulation of complement activation (RCA) locus" refers to a region of DNA sequence located on chromosome one that extends from the complement factor H (CFH) gene through the CD46 gene (also known as the MCP gene). The RCA locus comprises the CFH gene, the complement factor H related 3 (FHR3; also known as CFHR3, HFL4, and CFHL3) gene, the complement factor H related 1 (FHR1; also known as CHFR1, HRL1, HFL1, and CFHL1) gene, the complement factor H related 4 (FHR4; also known as CHFR4, CFHL4, which includes FHR4a and FHR4b splice variants) gene, the complement factor H related 2 (FHR2; also known as CHFR2, FHR2, HFL3, and CFHL2) gene, the complement factor H related 5 (FHR5; also known as CHFR5 and CFHL5) gene, and the complement factor 13B (F13B) gene, and is inclusive of the promoter regions of each gene, and non-genic and/or intergenic regions from at least 5 Kb, at least 10 Kb, at least 20 Kb to about 50 Kb upstream of CFH to at least 5 Kb, at least 10 Kb, at least 20 Kb to about 50 Kb downstream of F13B (*See* Figure 1). It is understood in the art that regulatory regions for a gene, such as enhances or repressors, can be identified at significant distances both proximal and distal to the transcriptional start site. Gene identifiers based on the EnsEMBL database are provided in Table V for each genes within the RCA locus described herein.

### II. Introduction

A study was conducted to elucidate potential associations between complement system genes (e.g., genes within the regulation of complement activation (RCA) locus including CFH, FHR3, FHR1, FHR4, FHR2, FHR5, and F13B) and other selected genes with age-related macular degeneration (AMD) and membranoproliferative glomerulonephritis type II (MPGNII). The associations discovered form the basis of the present disclosure, which provides methods for identifying individuals at increased risk, or at decreased risk, relative to the general population for a complement-related disease such as AMD and MPGNII. Further disclosed are kits, reagents and devices useful for making such determinations. The methods and reagents disclosed herein are also useful for determining prognosis.

### Use of polymorphisms to detect risk and protection

Disclosed is a method for detecting an individual's increased or decreased risk for development of progression of a complement-related disease such as AMD and MPGNII by detecting the presence of certain polymorphisms present in the individual's genome that are informative of his or her future disease status (including prognosis and appearance of signs of disease). The presence of such a polymorphism can be regarded as indicative of increased or decreased risk for the disease, especially in individuals who lack other predisposing or protective polymorphisms for the same disease(s). Even in cases where the predictive contribution of a given polymorphism is relatively minor by itself, genotyping contributes information that nevertheless can be useful for a characterization of an individual's predisposition to developing a disease. The information can be particularly useful when combined with genotype information from other loci *(e.g.,* the presence of a certain polymorphism may be more predictive or informative when used in combination with at least one other polymorphism).

### III. New SNPs associated with propensity to develop disease

In order to identify new single nucleotide polymorphisms (SNPs) associated with increased or decreased risk of developing complement-related diseases such as age-related macular degeneration (AMD) and MPGNII, 74 complement pathway-associated genes (and a number of inflammation-associated genes including toll-like receptors, or TLRs) were selected for SNP discovery. New SNPs in the candidate genes were discovered from a pool of 475 DNA samples derived from study participants with a history of AMD using a multiplexed SNP enrichment technology called Mismatch Repair Detection (ParAllele Biosciences/Affymetrix), an approach that enriches for variants from pooled samples. This SNP discovery phase (also referred to herein as Phase I) was conducted using DNA derived solely from individuals with AMD based upon the rationale that the discovered SNPs might be highly relevant to disease (e.g., AMD-associated).

### IV. Association of SNPs and Complement-Related Conditions

In Phase II of the study, 1162 DNA samples were employed for genotyping known and newly discovered SNPs in 340 genes. Genes investigated in Phase II included the complement and inflammation-associated genes used for SNP Discovery (Phase I). The remaining genes were selected based upon a tiered strategy, which was designed as follows. Genes received the highest priority if they fell within an AMD-harboring locus established by genome-wide linkage analysis or conventional linkage, or if they were differentially expressed at the RPE-choroid interface in donors with AMD compared to donors without AMD. Particular attention was paid to genes known to participate in inflammation, immune-associated processes, coagulation/fibrinolysis and/or extracellular matrix homeostasis.

In choosing SNPs for these genes, a higher SNP density in the genic regions, which was defined as 5Kb upstream from the start of transcription until 5Kb downstream from the end of transcription, was applied. In these regions, an average density of 1 SNP per 10Kb was used. In the non-genic regions of clusters of complement-related genes, an average of 1 SNP per 20 Kb was employed. The SNPs were chosen from HapMap data in the Caucasian population, the SNP Consortium (Marshall [1999] Science 284[5413]: 406-407), Whitehead, NCBI and the Celera SNP database. Selection included intronic SNPs, variants from the regulatory regions (mainly promoters) and coding SNPs (cSNPs) included in open reading frames. Data obtained by direct screening were used to validate the information extracted from databases. Thus, the overall sequence variation of functionally important regions of candidate genes was investigated, not only on a few polymorphisms using a previously described algorithm for tag selection.

Positive controls included CEPH members (*i*.*e*., DNA samples derived from lymphoblastoid cell lines from 61 reference families provided to the NIGMS Repository by the Centre de'Etude du Polymorphism Humain (CEPH), Foundation Jean Dausset in Paris, France) of the HapMap trios; the nomenclature used for these samples is the Coriell sample name (*i.e.,* family relationships were verified by the Coriell Institute for Medical Research Institute for Medical Research). The panel also contained a limited number of X-chromosome probes from two regions. These were included to provide additional information for inferring sample sex. Specifically, if the sample is clearly heterozygous for any X-chromosome markers, it must have two X-chromosomes. However, because there are a limited number of X-chromosome markers in the panel, and because their physical proximity likely means that there are even fewer haplotypes for these markers, we expected that samples with two X-chromosomes might also genotype as homozygous for these markers. The standard procedure for checking sample concordance involved two steps. The first step was to compare all samples with identical names for repeatability. In this study, the only repeats were positive controls and those had repeatability greater than 99.3% (range 99.85% to 100%). The second step was to compare all unique samples to all other unique samples and identify highly concordant sample pairs. Highly concordant sample pairs were used to identify possible tracking errors. The concordance test resulted in 20 sample pairs with concordance greater than 99%.

Samples were genotyped using multiplexed Molecular Inversion Probe (MIP) technology (ParAllele Biosciences/Affymetrix). Successful genotypes were obtained for 3,267 SNPs in 347 genes in 1113 unique samples (out of 1162 unique submitted samples; 3,267 successful assays out 3,308 assays attempted). SNPs with more than 5% failed calls (45 SNPs), SNPs with no allelic variation (354 alleles) and subjects with more than 5% missing genotypes (11 subjects) were deleted.

The resulting genotype data were analyzed in multiple sub-analyses, using a variety of appropriate statistical analyses, as described below.

### A. Polymorphisms associated with AMD:

One genotype association analysis was performed on all SNPs comparing samples derived from individuals with AMD to those derived from an ethnic- and age-matched control cohort. All genotype associations were assessed using a statistical software program known as SAS®. SNPs showing significant association with AMD are shown in Table I and Table IA. Table I and Table IA include SNPs from FHR1, FHR2, FHR4, FHR5, and F13B, with additional raw data provided in Table III as discussed in greater detail hereinbelow. The genotypes depicted in Tables I and IA are organized alphabetically by gene symbol. AMD associated SNPs identified in a given gene are designated by SNP number or MRD designation. For each SNP, allele frequencies are shown as percentages in both control and disease (AMD) populations. Allele frequencies are provided for individuals homozygous for allele 1 and allele 2, and for heterozygous individuals. For example, for SNP rs5997, which is located in complement factor 13B (F13B), 1% of the control population is homozygous for allele 1 (*i*.*e*., the individual has a "A" base at this position), 77.9% of the control population is homozygous for allele 2 (*i*.*e*., the individual has a "G" base at this position), and 21% of the control population is heterozygous. The overall frequency for allele 1 (*i*.*e*., the "A" allele) in the control population is 11.6% and the overall frequency for allele 2 in the control population is 88.4%. In the AMD population, 0.4% of the population is homozygous for allele 1 (the "A" allele), 90.1% of population is homozygous for allele 2 (the "G" allele), and 9.5% of the population is heterozygous. The overall frequency for allele 1 (the "A" allele) in the AMD population is 5.2% and the overall frequency for allele 2 (the "G" allele) in the AMD population is 94.8%. Genotype-likelihood ratio (3 categories; genotype p value) and Chi Square values ("Freq. Chi Square (both collapsed- 2 categories)") are provided for each SNP.

In some cases in Table I, "MRD" designations derived from discovered SNPs are provided in place of SNP number designations. MRD_3905 corresponds to the following sequence, which is the region flanking a SNP present in the FHR5 gene: TGCAGAAAAGGATGCGTGTGAACAGCAGGTA(A/G) TTTTCTTCTGATTGATTCTATATCTAGATGA (SEQ ID NO: 1). MRD_3906 corresponds to the following sequence, which is the region flanking the SNP present in the FHR5 gene: GGGGAAAAGCAGTGTGGAAATTATTTAGGAC(C/T)GTGTTCATTAATTTAAAGCA AGGCAAGTCAG (SEQ ID NO: 2). The polymorphic site indicating the SNP associated alleles are shown in parentheses. Further, certain SNPs presented in Table I were previously identified by MRD designations in provisional application, US Application No. 60/984,702. For example, the SNP designated rs1412631 is also called MRD_3922. The SNP designated rs12027476 is also called MRD_3863.

The presence in the genome or the transcriptome of an individual of one or more polymorphisms listed in Table I and/or Table IA is associated with an increased or decreased risk of AMD. Accordingly, detection of a polymorphism shown in Table I or Table IA in a nucleic acid sample of an individual can indicate that the individual is at increased risk for developing AMD. One of skill in the art will be able to refer to Table I or Table IA to identify alleles associated with increased (or decreased) likelihood of developing AMD. For example, in the gene F13B, allele 2 of the SNP rs5997 is found in 94.8% of AMD chromosomes, but only in 88.4% of the control chromosomes indicating that a person having allele 2 has a greater likelihood of developing AMD than a person not having allele 2 (*See* Table I). Allele 2 ("G") is the more common allele (*i.e.* the "wild type" allele). The "A" allele is the rarer allele, but is more prevalent in the control population than in the AMD population: it is therefore a "protective polymorphism." Table III(A-B) provides the raw data from which the percentages of allele frequencies as shown in Tables I and IA were calculated. Table III(C) depicts the difference in percentage allele frequency in homozygotes for allele 1 and allele 2 between control and disease populations, the difference in percentage allele frequency in heterozygotes between control and disease populations, and the difference in percentage for undetermined subjects between control and disease populations. Table VI provides the nucleotide sequences flanking the SNPs disclosed in Tables I and IA. For each sequence, the "N" refers to the polymorphic site. The nucleotide present at the polymorphic site is either allele 1 or allele 2 as shown in Table I and Table IA.

In other embodiments, the presence of a combination of multiple (e.g., two or more, or three or more, four or more, or five or more) AMD-associated polymorphisms shown in Table I and/or Table IA indicates an increased (or decreased) risk for AMD.

In addition to the new AMD SNP associations defined herein, these experiments confirmed previously reported associations of AMD with variations/SNPs in the CFH, FHR1-5, F13B, LOC387715, PLEKHA1 and PRSS11 genes.

### B. Polymorphisms associated with MPGNII

Another genotype association analysis was performed on all SNPs comparing samples derived from MPGNII cases to those derived from an age-matched control cohort. Genotypes containing SNPs showing significant association with MPGNII are shown in Table II. As described above for Tables I and IA, the genotypes depicted in Table II are organized alphabetically by gene symbol. MPGNII associated SNPs identified in a given gene are designated by SNP number. For each SNP, allele frequencies are presented as percentages in both control and disease (MPGNII) populations. Allele frequencies are shown for homozygous individuals for allele 1 and allele 2, and heterozygous individuals. Genotype likelihood ratios (genotype p value), Chi Square values, and Fisher Exact Test values are provided for each SNP.

The presence of one or more polymorphisms listed in Tables II is associated with an increased or decreased risk of MPGNII. Accordingly, the presence of a polymorphism shown in Table II in a nucleic acid sample of an individual can indicate that the individual is at increased risk for developing MPGNII. One of skill in the art will be able to refer to Tables II to identify alleles associated with increased (or decreased) likelihood of developing MPGNII. For example, in the gene CFH, allele 1 of the SNP rs3753395 is found in 92.1% of MPGNII chromosomes, indicating that a person having allele 1 has a greater likelihood of developing MPGNII than a person not having allele 1 (58.6% *-See* Table II). Allele 1 ("A") is the more common allele (*i.e.* the "wild type" allele). The "T" allele is the rarer allele, but is more prevalent in the control population than in the MPGNII population: it is therefore a "protective polymorphism." Table IV(A-B) provides the raw data from which the percentages of allele frequencies as shown in Table II were calculated. Table IV(C) depicts the difference in percentage allele frequency in homozygotes for allele 1 and allele 2 between control and disease populations, the difference in percentage allele frequency in heterozygotes between control and disease populations, and the difference in percentage for undetermined subjects between control and disease populations. Table VII provides the nucleotide sequences flanking the SNPs disclosed in Table II. For each sequence, the "N" refers to the polymorphic site. The nucleotide present at the polymorphic site is either allele 1 or allele 2 as shown in Table II.

In other embodiments, the presence of a combination of multiple (e.g., two or more, or three or more) MPGNII-associated polymorphisms shown in Table II indicates an increased (or decreased) risk for MPGNII.

### V. Determination of Risk (Screening):

### Determining the risk of an individual

An individual's relative risk *(i.e.,* susceptibility or propensity) of developing a particular complement-related disease characterized by dysregulation of the complement system can be determined by screening for the presence or absence of a genetic profile in the regulation of complement activation (RCA) locus of chromosome one. In a preferred embodiment, the complement-related disease characterized by complement dysregulation is AMD and/or MPGNII.

A genetic profile for AMD comprises one or more single nucleotide polymorphisms (SNPs) selected from Table I and/or Table IA. The presence of any one of the SNPs listed in Table I or Table IA is informative (*i.e.,* indicative) of an individual's increased or decreased risk of developing AMD or for predicting the course of progression of AMD in the individual (*i.e.,* a patient).

The predictive value of a genetic profile for AMD can be increased by screening for a combination of SNPs selected from Table I and/or Table IA. In one embodiment, the predictive value of a genetic profile is increased by screening for the presence of at least 2 SNPs, at least 3 SNPs, at least 4 SNPs, at least 5 SNPs, at least 6 SNPs, at least 7 SNPs, at least 8 SNPS, at least 9 SNPs, or at least 10 SNPs selected from Table I and/or Table IA. In another embodiment, the predictive value of a genetic profile for AMD is increased by screening for the presence of at least one SNP from Table I and/or Table IA and at least one additional SNP selected from the group consisting of a polymorphism in exon 22 of CFH (R1210C), rs1061170, rs203674, rs1061147, rs2274700, rs12097550, rs203674, rs9427661, rs9427662, rs10490924, rs11200638, rs2230199, rs800292, rs3766404, rs529825, rs641153, rs4151667, rs547154, rs9332739, rs2511989, rs3753395, rs1410996, rs393955, rs403846, rs1329421, rs10801554, rs12144939, rs12124794, rs2284664, rs16840422, and rs6695321. In certain embodiments, the method may comprise screening for at least one SNP from Table I or Table IA and at least one additional SNP associated with risk of AMD selected from the group consisting of: a polymorphism in exon 22 of CFH (R1210C), rs1061170, rs203674, rs1061147, rs2274700, rs12097550, rs203674, rs9427661, rs9427662, rs10490924, rs11200638, and rs2230199.

The predictive value of a genetic profile for AMD can also be increased by screening for a combination of predisposing and protective polymorphisms. For example, the absence of at least one, typically multiple, predisposing polymorphisms and the presence of at least one, typically multiple, protective polymorphisms may indicate that the individual is not at risk of developing AMD. Alternatively, the presence of at least one, typically multiple, predisposing SNPs and the absence of at least one, typically multiple, protective SNPs indicate that the individual is at risk of developing AMD. In one embodiment, a genetic profile for AMD comprises screening for the presence of at least one SNP selected from Table I or Table IA and the presence or absence of at least one protective SNP selected from the group consisting of: rs800292, rs3766404, rs529825, rs641153, rs4151667, rs547154, and rs9332739.

In some embodiments, the genetic profile comprises at least one SNP in F13B. In one embodiment, the at least one SNP includes rs5997. In one embodiment, the at least one SNP includes rs6428380. In one embodiment, the at least one SNP includes rs1794006. In one embodiment, the at least one SNP includes rs10801586.

In some embodiments, the genetic profile comprises at least one SNP in FHR1. In one embodiment, the at least one SNP includes rs12027476. In one embodiment, the at least one SNP includes rs436719.

In some embodiments, the genetic profile comprises at least one SNP in FHR2. In one embodiment, the at least one SNP includes rs12066959. In one embodiment, the at least one SNP includes rs3828032. In one embodiment, the at least one SNP includes rs6674522. In one embodiment, the at least one SNP includes rs432366.

In some embodiments, the genetic profile comprises at least one SNP in FHR4. In one embodiment, the at least one SNP includes rs1409153.

In some embodiments, the genetic profile comprises at least one SNP in FHR5. In one embodiment, the at least one SNP includes MRD_3905. In one embodiment, the at least one SNP includes MRD_3906. In one embodiment, the at least one SNP includes rs10922153.

Although the predictive value of the genetic profile can generally be enhanced by the inclusion of multiple SNPs, no one of the SNPs is indispensable. Accordingly, in various embodiments, one or more of the SNPs is omitted from the genetic profile.

In certain embodiments, the genetic profile comprises a combination of at least two SNPs selected from the pairs identified below:

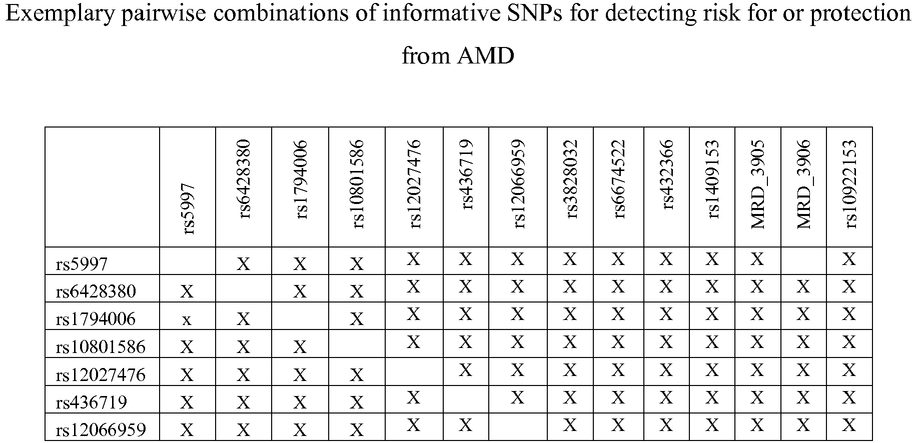

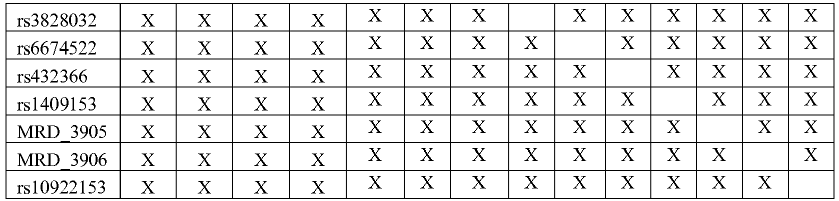

In a further embodiment, the determination of an individual's genetic profile can include screening for a deletion or a heterozygous deletion within the RCA locus that is associated with AMD risk or protection. Exemplary deletions that are associated with AMD protection include deletion of FHR3 and FHR1 genes. The deletion may encompass one gene, multiple genes, a portion of a gene, or an intergenic region, for example. If the deletion impacts the size, conformation, expression or stability of an encoded protein, the deletion can be detected by assaying the protein, or by querying the nucleic acid sequence of the genome or transcriptome of the individual.

A genetic profile for MPGNII comprises one or more single nucleotide polymorphisms selected from Table II. The presence of any one of the SNPs listed in Table II is informative of an individual's increased risk of developing MPGNII or for predicting the course of progression of MPGNII in the individual (*i.e.,* a patient).

The predictive value of a genetic profile for MPGNII can be increased by screening for a combination of predisposing single nucleotide polymorphisms. In one embodiment, the predictive value of a genetic profile is increased by screening for the presence of at least 2 SNPs, at least 3 SNPs, at least 4 SNPs, at least 5 SNPs, at least 6 SNPs, at least 7 SNPs, at least 8 SNPS, at least 9 SNPs, or at least 10 SNPs selected from Table II. In another embodiment, the predictive value of a genetic profile for MPGNII is increased by screening for the presence of at least one SNP from Table II and at least one additional SNP selected from the group consisting of a polymorphism in exon 22 of CFH (R1210C), rs1061170, rs203674, rs1061147, rs2274700, rs12097550, rs203674, rs9427661, rs9427662, rs10490924, rs11200638, rs2230199, rs800292, rs3766404, rs529825, rs641153, rs4151667, rs547154, and rs9332739. In an exemplary embodiment, the at least one additional SNP is selected from the group consisting of rs1061170, rs12097550, rs9427661, and rs9427662.

The predictive value of a genetic profile for MPGNII can also be increased by screening for a combination of predisposing and protective polymorphisms. For example, the absence of predisposing SNPs and the presence of a protective polymorphisms indicates that the individual is not at risk of developing MPGNII. Alternatively, the presence of a predisposing SNP and the absence of a protective SNP indicates that the individual is at risk of developing MPGNII. In one embodiment, a genetic profile for MPGNII comprises screening for the presence of at least one SNP selected from Table II and the presence of at least one protective SNP selected from the group consisting of rs800292, rs3766404, rs529825, rs641153, rs4151667, rs547154, rs9332739, and rs2274700. In an exemplary embodiment, the at least one protective SNP is selected from the group consisting of rs800292, rs3766404, rs529825, and rs2274700.

In some embodiments, the genetic profile comprises at least one SNP in CFH. In one embodiment, the at least one SNP includes rs3753395. In one embodiment, the at least one SNP includes rs1410996. In one embodiment, the at least one SNP includes rs1329421. In one embodiment, the at least one SNP includes rs10801554. In one embodiment, the at least one SNP includes rs12124794. In one embodiment, the at least one SNP includes rs393955. In one embodiment, the at least one SNP includes rs403846. In one embodiment, the at least one SNP includes rs2284664. In one embodiment, the at least one SNP includes rs12144939.

In some embodiments, the genetic profile comprises at least one SNP in F13B. In one embodiment, the at least one SNP includes rs2990510.

In some embodiments, the genetic profile comprises at least one SNP in FHR1. In one embodiment, the at least one SNP includes rs12027476.

In some embodiments, the genetic profile comprises at least one SNP in FHR2. In one embodiment, the at least one SNP includes rs12066959. In one embodiment, the at least one SNP includes rs4085749.

In some embodiments, the genetic profile comprises at least one SNP in FHR4. In one embodiment, the at least one SNP includes rs1409153.

In certain embodiments, the genetic profile comprises a combination of at least two SNPs selected from the pairs identified below:

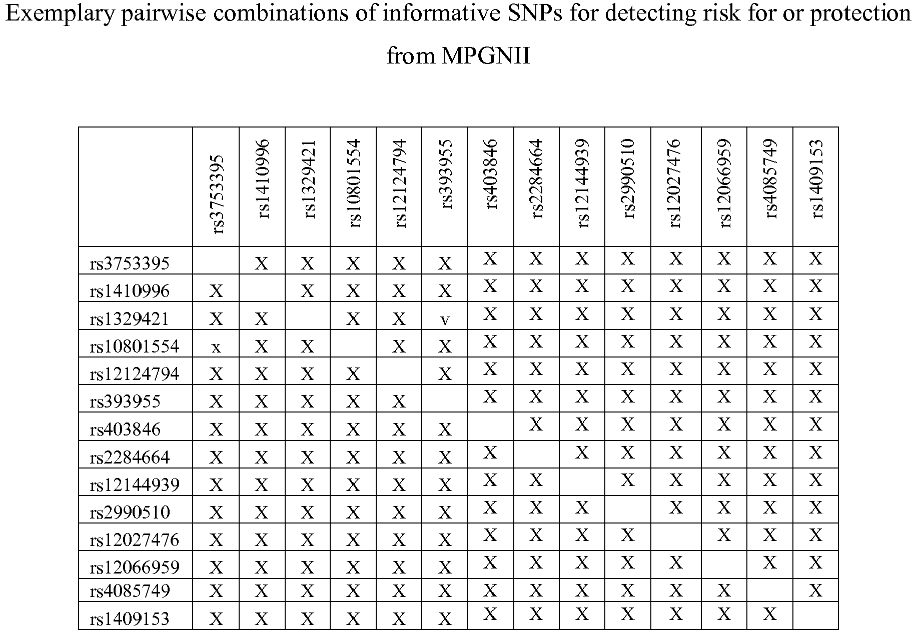

Further, determining an individual's genetic profile may include determining an individual's genotype or haplotype to determine if the individual is at an increased or decreased risk of developing AMD and/or MPGNII. In one embodiment, an individual's genetic profile may comprise SNPs that are in linkage disequilibrium with other SNPs associated with AMD and/or MPGNII that define a haplotype (*i*.*e*., a set of polymorphisms in the RCA locus) associated with risk or protection of AMD and/or MPGNII. In another embodiment, a genetic profile may include multiple haplotypes present in the genome or a combination of haplotypes and polymorphisms, such as single nucleotide polymorphisms, in the genome, *e.g.,* a haplotype in the RCA locus and a haplotype or at least one SNP on chromosome 10.

Further studies of the identity of the various SNPs and other genetic characteristics disclosed herein with additional cohorts, and clinical experience with the practice of this invention on patient populations, will permit ever more precise assessment of AMD or MPGN-II risk bases on emergent SNP patterns. This work will result in refinement of which particular set of SNPs are characteristic of a genetic profile which is, for example, indicative of an urgent need for intervention, or indicative that the early stages of AMD observed in a individual is unlikely to progress to more serious disease, or is likely to progress rapidly to the wet form of the disease, or that the presenting individual is not at significant risk of developing AMD, or that a particular AMD therapy is most likely to be successful with this individual and another therapeutic alternative less likely to be productive. Thus, it is anticipated that the practice of the invention disclosed herein, especially when combined with the practice of risk assessment using other known risk-indicative and protection-indicative SNPs, will permit disease management and avoidance with increasing precision.

A single nucleotide polymorphism comprised within a genetic profile for AMD and/or MPGNII as described herein may be detected directly or indirectly. Direct detection refers to determining the presence or absence of a specific SNP identified in the genetic profile using a suitable nucleic acid, such as an oligonucleotide in the form of a probe or primer as described below. Alternatively, direct detection can include querying a pre-produced database comprising all or part of the individual's genome for a specific SNP in the genetic profile. Other direct methods are known to those skilled in the art. Indirect detection refers to determining the presence or absence of a specific SNP identified in the genetic profile by detecting a surrogate or proxy SNP that is in linkage disequilibrium with the SNP in the individual's genetic profile. Detection of a proxy SNP is indicative of a SNP of interest and is increasingly informative to the extent that the SNPs are in linkage disequilibrium, e.g., at least 50%, 60%, 70%, 80%, 90%, 95%, 98%, or about 100% LD. Another indirect method involves detecting allelic variants of proteins accessible in a sample from an individual that are consequent of a risk-associated or protection-associated allele in DNA that alters a codon.

It is also understood that a genetic profile as described herein may comprise one or more nucleotide polymorphism(s) that are in linkage disequilibrium with a polymorphism that is causative of disease. In this case, the SNP in the genetic profile is a surrogate SNP for the causative polymorphism.

Genetically linked SNPs, including surrogate or proxy SNPs, can be identified by methods known in the art. Non-random associations between polymorphisms (including single nucleotide polymorphisms, or SNPs) at two or more loci are measured by the degree of linkage disequilibrium (LD). The degree of linkage disequilibrium is influenced by a number of factors including genetic linkage, the rate of recombination, the rate of mutation, random drift, non-random mating and population structure. Moreover, loci that are in LD do not have to be located on the same chromosome, although most typically they occur as clusters of adjacent variations within a restricted segment of DNA. Polymorphisms that are in complete or close LD with a particular disease-associated SNP are also useful for screening, diagnosis, and the like.

SNPs in LD with each other can be identified using methods known in the art and SNP databases (e.g., the Perlegen database, at http://genome.perlegen.com/browser/download.html and others). For illustration, SNPs in linkage disequilibrium (LD) with the *CFH* SNP rs800292 were identified using the Perlegen database. This database groups SNPs into LD bins such that all SNPs in the bin are highly correlated to each other. For example, AMD-associated SNP rs800292 was identified in the Perlegen database under the identifier 'afd0678310'. A LD bin (European LD bin #1003371; see table below) was then identified that contained linked SNPs -- including afd1152252, afd4609785, afd4270948, afd0678315, afd0678311, and afd0678310 -- and annotations.

| **SNP ID** | | **SNP Position** | | | **Alleles** | **Allele Frequency** |
|---|---|---|---|---|---|---|
| **Perlegen 'afd' ID*** | **'ss' ID** | **Chromosome** | **Accession** | **Position** | | **European American** |
| afd1152252 | ss23875287 | 1 | NC_000001.5 | 193872580 | A/G | 0.21 |
| afd4609785 | ss23849009 | 1 | NC_000001.5 | 193903455 | G/A | 0.79 |
| afd4270948 | ss23849019 | 1 | NC_000001.5 | 193905168 | T/C | 0.79 |
| afd0678315 | ss23857746 | 1 | NC_000001.5 | 193923365 | G/A | 0.79 |
| afd0678311 | ss23857767 | 1 | NC_000001.5 | 193930331 | C/T | 0.79 |
| afd0678310 | ss23857774 | 1 | NC_000001.5 | 193930492 | G/A | 0.79 |

| | | | | | | |
|---|---|---|---|---|---|---|
| *Perlegen AFD identification numbers can be converted into conventional SNP database identifiers (in this case, rs4657825, rs576258, rs481595, rs529825, rs551397, and rs800292) using the NCBI database (http://www.ncbi.nlm.nih.gov/sites/entrez?db=snp&cmd=search&term=). | | | | | | |

Also, for illustration, SNPs in linkage disequilibrium (LD) with the *C4BPA* SNP rs2491395 were identified using the Perlegen database. This database groups SNPs into LD bins such that all SNPs in the bin are highly correlated to each other. For example, DDD-associated SNP rs2491395 was identified in the Perlegen database under its 'afd' identifier. A LD bin (see table below) was then identified that contained linked SNPs -- including afd1168850, afd1168843, afd1168839, afd1168834, and afd1168832 -- and annotations.

| **C4BP SNP ID** | | **SNP Position** | | | **Alleles** | **Allele Frequency** |
|---|---|---|---|---|---|---|
| **Perlegen 'afd' ID*** | **ss ID** | **Chromosome** | **Accession** | **Position** | | **European American** |
| afd1168850 | ss23669009 | 1 | NC_000001.5 | 204383958 | A/G | 0.71 |
| afd1168843 | ss24141938 | 1 | NC_000001.5 | 204385422 | T/A | 0.75 |
| afd1168839 | ss24617443 | 1 | NC_000001.5 | 204388599 | T/C | 0.69 |
| afd1168834 | ss23669012 | 1 | NC_000001.5 | 204389287 | C/T | 0.71 |
| afd1168832 | ss23669013 | 1 | NC_000001.5 | 204389369 | G/A | 0.69 |

| | | | | | | |
|---|---|---|---|---|---|---|
| *Perlegen AFD identification numbers can be converted into conventional SNP database identifiers (in this case, rs2491393, rs2491395, rs4844573, rs4571969, and rs4266889) using the NCBI database (http://www.ncbi.nlm.nih.gov/sites/entrez?db=snp&cmd=search&term=). | | | | | | |

The frequencies of these alleles in disease versus control populations may be determined using the methods described herein.

As a second example, the LD tables computed by HapMap were downloaded (http://ftp.hapmap.org/ld_data/latest/). Unlike the Perlegen database, the HapMap tables use 'rs' SNP identifiers directly. All SNPs with an R² value greater than 0.80 when compared to rs800292 were extracted from the database in this illustration. Due to the alternate threshold used to compare SNPs and the greater SNP coverage of the HapMap data, more SNPs were identified using the HapMap data than the Perlegen data.

| **SNP 1 Location** | **SNP #2 Location** | **Population** | **SNP #1 ID** | **SNP #2 ID** | **D'** | **R²** | **LOD** |
|---|---|---|---|---|---|---|---|
| 194846662 | 194908856 | CEU | rs10801551 | rs800292 | 1 | 0.84 | 19.31 |
| 194850944 | 194908856 | CEU | rs4657825 | rs800292 | 1 | 0.9 | 21.22 |
| 194851091 | 194908856 | CEU | rs12061508 | rs800292 | 1 | 0.83 | 18.15 |
| 194886125 | 194908856 | CEU | rs505102 | rs800292 | 1 | 0.95 | 23.04 |
| 194899093 | 194908856 | CEU | rs6680396 | rs800292 | 1 | 0.84 | 19.61 |
| 194901729 | 194908856 | CEU | rs529825 | rs800292 | 1 | 0.95 | 23.04 |
| 194908856 | 194928161 | CEU | rs800292 | rs12124794 | 1 | 0.84 | 18.81 |
| 194908856 | 194947437 | CEU | rs800292 | rs1831281 | 1 | 0.84 | 19.61 |
| 194908856 | 194969148 | CEU | rs800292 | rs2284664 | 1 | 0.84 | 19.61 |
| 194908856 | 194981223 | CEU | rs800292 | rs10801560 | 1 | 0.84 | 19.61 |
| 194908856 | 194981293 | CEU | rs800292 | rs10801561 | 1 | 0.84 | 19.61 |
| 194908856 | 195089923 | CEU | rs800292 | rs10922144 | 1 | 0.84 | 19.61 |

As indicated above, publicly available databases such as the HapMap database (http://ftp.hapmap.org/ld_data/latest/) and Haploview (Barrett, J.C. et al., Bioinformatics 21, 263 (2005)) may be used to calculate linkage disequilibiurm between two SNPs. The frequency of identified alleles in disease versus control populations may be determined using the methods described herein. Statistical analyses may be employed to determine the significance of a non-random association between the two SNPs (e.g., Hardy-Weinberg Equilibrium, Genotype likelihood ratio (genotype p value), Chi Square analysis, Fishers Exact test). A statistically significant non-random association between the two SNPs indicates that they are in linkage disequilibrium and that one SNP can serve as a proxy for the second SNP.

The screening step to determine an individual's genetic profile may be conducted by inspecting a data set indicative of genetic characteristics previously derived from analysis of the individual's genome. A data set indicative of an individual's genetic characteristics may include a complete or partial sequence of the individual's genomic DNA, or a SNP map. Inspection of the data set including all or part of the individual's genome may optimally be performed by computer inspection. Screening may further comprise the step of producing a report identifying the individual and the identity of alleles at the site of at least one or more polymorphisms shown in Table I, Table IA or Table II and/or proxy SNPs.

Alternatively, the screening step to determine an individual's genetic profile comprises analyzing a nucleic acid (*i.e.,* DNA or RNA) sample obtained from the individual. A sample can be from any source containing nucleic acids (e.g., DNA or RNA) including tissues such as hair, skin, blood, biopsies of the retina, kidney, or liver or other organs or tissues, or sources such as saliva, cheek scrapings, urine, amniotic fluid or CVS samples, and the like. Typically, genomic DNA is analyzed. Alternatively, RNA, cDNA, or protein can be analyzed. Methods for the purification or partial purification of nucleic acids or proteins from an individual's sample, and various protocols for analyzing samples for use in diagnostic assays are well known.

A polymorphism such as a SNP can be conveniently detected using suitable nucleic acids, such as oligonucleotides in the form of primers or probes. Accordingly, the disclosure not only provides novel SNPs and/or novel combinations of SNPs that are useful in assessing risk for a complement-related disease, but also nucleic acids such as oligonucleotides useful to detect them. A useful oligonucleotide for instance comprises a sequence that hybridizes under stringent hybridization conditions to at least one polymorphism identified herein. Where appropriate, at least one oligonucleotide comprises a sequence that is fully complementary to a nucleic acid sequence comprising at least one polymorphism identified herein. Such oligonucleotide(s) can be used to detect the presence of the corresponding polymorphism, for example by hybridizing to the polymorphism under stringent hybridizing conditions, or by acting as an extension primer in either an amplification reaction such as PCR or a sequencing reaction, wherein the corresponding polymorphism is detected either by amplification or sequencing. Suitable detection methods are described below.

An individual's genotype can be determined using any method capable of identifying nucleotide variation, for instance at single nucleotide polymorphic sites. The particular method used is not a critical aspect of the invention. Although considerations of performance, cost, and convenience will make particular methods more desirable than others, it will be clear that any method that can detect one or more polymorphisms of interest can be used to practice the invention. A number of suitable methods are described below.

### 1) Nucleic acid analysis

### General

Polymorphisms can be identified through the analysis of the nucleic acid sequence present at one or more of the polymorphic sites. A number of such methods are known in the art. Some such methods can involve hybridization, for instance with probes (probe-based methods). Other methods can involve amplification of nucleic acid (amplification-based methods). Still other methods can include both hybridization and amplification, or neither.

### a) Amplification-based methods

### Preamplification followed by sequence analysis:

Where useful, an amplification product that encompasses a locus of interest can be generated from a nucleic acid sample. The specific polymorphism present at the locus is then determined by further analysis of the amplification product, for instance by methods described below. Allele-independent amplification can be achieved using primers which hybridize to conserved regions of the genes. The genes contain many invariant or monomorphic regions and suitable allele-independent primers can be selected routinely.

Upon generation of an amplified product, polymorphisms of interest can be identified by DNA sequencing methods, such as the chain termination method (Sanger et al., 1977, Proc. Natl. Acad. Sci,. 74:5463-5467) or PCR-based sequencing. Other useful analytical techniques that can detect the presence of a polymorphism in the amplified product include single-strand conformation polymorphism (SSCP) analysis, denaturing gradient gel electropohoresis (DGGE) analysis, and/or denaturing high performance liquid chromatography (DHPLC) analysis. In such techniques, different alleles can be identified based on sequence- and structure-dependent electrophoretic migration of single stranded PCR products. Amplified PCR products can be generated according to standard protocols, and heated or otherwise denatured to form single stranded products, which may refold or form secondary structures that are partially dependent on base sequence. An alternative method, referred to herein as a kinetic-PCR method, in which the generation of amplified nucleic acid is detected by monitoring the increase in the total amount of double-stranded DNA in the reaction mixture, is described in Higuchi et al., 1992, Bio/Technology, 10:413-417.

### Allele-specific amplification:

Alleles can also be identified using amplification-based methods. Various nucleic acid amplification methods known in the art can be used in to detect nucleotide changes in a target nucleic acid. Alleles can also be identified using allele-specific amplification or primer extension methods, in which amplification or extension primers and/or conditions are selected that generate a product only if a polymorphism of interest is present.

### Amplification technologies

A preferred method is the polymerase chain reaction (PCR), which is now well known in the art, and described in U.S. Pat. Nos. 4,683,195; 4,683,202; and 4,965,188. Other suitable amplification methods include the ligase chain reaction (Wu and Wallace, 1988, Genomics 4:560-569); the strand displacement assay (Walker et al., 1992, Proc. Natl. Acad. Sci. USA 89:392-396, Walker et al. 1992, Nucleic Acids Res. 20:1691-1696, and U.S. Pat. No. 5,455,166); and several transcription-based amplification systems, including the methods described in U.S. Pat. Nos. 5,437,990; 5,409,818; and 5,399,491; the transcription amplification system (TAS) (Kwoh et al., 1989, Proc. Natl. Acad. Sci. USA, 86:1173-1177); and self-sustained sequence replication (3SR) (Guatelli et al., 1990, Proc. Natl. Acad. Sci. USA, 87:1874-1878 and WO 92/08800). Alternatively, methods that amplify the probe to detectable levels can be used, such as QB-replicase amplification (Kramer et al., 1989, Nature, 339:401-402, and Lomeli et al., 1989, Clin. Chem., 35:1826-1831). A review of known amplification methods is provided in Abramson et al., 1993, Current Opinion in Biotechnology, 4:41-47.

### Amplification of mRNA

Genotyping also can also be carried out by detecting and analyzing mRNA under conditions when both maternal and paternal chromosomes are transcribed. Amplification of RNA can be carried out by first reverse-transcribing the target RNA using, for example, a viral reverse transcriptase, and then amplifying the resulting cDNA, or using a combined high-temperature reverse-transcription-polymerase chain reaction (RT-PCR), as described in U.S. Pat. Nos. 5,310,652; 5,322,770; 5,561,058; 5,641,864; and 5,693,517 (*see* also Myers and Sigua, 1995, in PCR Strategies, supra, chapter 5).

### Selection of allele-specific primers

The design of an allele-specific primer can utilize the inhibitory effect of a terminal primer mismatch on the ability of a DNA polymerase to extend the primer. To detect an allele sequence using an allele-specific amplification or extension-based method, a primer complementary to the genes of interest is chosen such that the nucleotide hybridizes at or near the polymorphic position. For instance, the primer can be designed to exactly match the polymorphism at the 3' terminus such that the primer can only be extended efficiently under stringent hybridization conditions in the presence of nucleic acid that contains the polymorphism. Allele-specific amplification- or extension-based methods are described in, for example, U.S. Pat. Nos. 5,137,806; 5,595,890; 5,639,611; and U.S. Pat. No. 4,851,331.

### Analysis of heterozygous samples

If so desired, allele-specific amplification can be used to amplify a region encompassing multiple polymorphic sites from only one of the two alleles in a heterozygous sample.

### b) Probe-based methods:

### General

Alleles can be also identified using probe-based methods, which rely on the difference in stability of hybridization duplexes formed between a probe and its corresponding target sequence comprising an allele. For example, differential probes can be designed such that under sufficiently stringent hybridization conditions, stable duplexes are formed only between the probe and its target allele sequence, but not between the probe and other allele sequences.

### Probe design

A suitable probe for instance contains a hybridizing region that is either substantially complementary or exactly complementary to a target region of a polymorphism described herein or their complement, wherein the target region encompasses the polymorphic site. The probe is typically exactly complementary to one of the two allele sequences at the polymorphic site. Suitable probes and/or hybridization conditions, which depend on the exact size and sequence of the probe, can be selected using the guidance provided herein and well known in the art. The use of oligonucleotide probes to detect nucleotide variations including single base pair differences in sequence is described in, for example, Conner et al., 1983, Proc. Natl. Acad. Sci. USA, 80:278-282, and U.S. Pat. Nos. 5,468,613 and 5,604,099.

### Pre-amplification before probe hybridization

In an embodiment, at least one nucleic acid sequence encompassing one or more polymorphic sites of interest are amplified or extended, and the amplified or extended product is hybridized to one or more probes under sufficiently stringent hybridization conditions. The alleles present are inferred from the pattern of binding of the probes to the amplified target sequences.

### Some known probe-based genotyping assays

Probe-based genotyping can be carried out using a "TaqMan" or "5'-nuclease assay," as described in U.S. Pat. Nos. 5,210,015; 5,487,972; and 5,804,375; and Holland et al., 1988, Proc. Natl. Acad. Sci. USA, 88: 7276-7280. Examples of other techniques that can be used for SNP genotyping include, but are not limited to, Amplifluor, Dye Binding-Intercalation, Fluorescence Resonance Energy Transfer (FRET), Hybridization Signal Amplification Method (HSAM), HYB Probes, Invader/Cleavase Technology (Invader/CFLP), Molecular Beacons, Origen, DNA-Based Ramification Amplification (RAM), Rolling circle amplification (RCA), Scorpions, Strand displacement amplification (SDA), oligonucleotide ligation (Nickerson et al., Proc. Natl Acad. Sci. USA, 87: 8923-8927) and/or enzymatic cleavage. Popular high-throughput SNP-detection methods also include template-directed dye-terminator incorporation (TDI) assay (Chen and Kwok, 1997, Nucleic Acids Res. 25: 347-353), the 5'-nuclease allele-specific hybridization TaqMan assay (Livak et al. 1995, Nature Genet. 9: 341-342), and the recently described allele-specific molecular beacon assay (Tyagi et al. 1998, Nature Biotech. 16: 49-53).

### Assay formats

Suitable assay formats for detecting hybrids formed between probes and target nucleic acid sequences in a sample are known in the art and include the immobilized target (dot-blot) format and immobilized probe (reverse dot-blot or line-blot) assay formats. Dot blot and reverse dot blot assay formats are described in U.S. Pat. Nos. 5,310,893; 5,451,512; 5,468,613; and 5,604,099. In some embodiments multiple assays are conducted using a microfluidic format. *See, e.g.,* Unger et al., 2000, Science 288:113-6.

### Nucleic acids containing polymorphisms of interest

Further disclosed are isolated nucleic acid molecules, *e.g*., oligonucleotides, probes and primers, comprising a portion of the genes, their complements, or variants thereof as identified herein. Preferably the variant comprises or flanks at least one of the polymorphic sites identified herein, for example variants associated with AMD and/or MPGNII.

Nucleic acids such as primers or probes can be labeled to facilitate detection. Oligonucleotides can be labeled by incorporating a label detectable by spectroscopic, photochemical, biochemical, immunochemical, radiological, radiochemical or chemical means. Useful labels include ³²P, fluorescent dyes, electron-dense reagents, enzymes, biotin, or haptens and proteins for which antisera or monoclonal antibodies are available.

### 2) Protein-based or phenotypic detection of polymorphism:

Where polymorphisms are associated with a particular phenotype, then individuals that contain the polymorphism can be identified by checking for the associated phenotype. For example, where a polymorphism causes an alteration in the structure, sequence, expression and/or amount of a protein or gene product, and/or size of a protein or gene product, the polymorphism can be detected by protein-based assay methods.

### Techniques for protein analysis

Protein-based assay methods include electrophoresis (including capillary electrophoresis and one- and two-dimensional electrophoresis), chromatographic methods such as high performance liquid chromatography (HPLC), thin layer chromatography (TLC), hyperdiffusion chromatography, and mass spectrometry.

### Antibodies

Where the structure and/or sequence of a protein is changed by a polymorphism of interest, one or more antibodies that selectively bind to the altered form of the protein can be used. Such antibodies can be generated and employed in detection assays such as fluid or gel precipitin reactions, immunodiffusion (single or double), immunoelectrophoresis, radioimmnunoassay (RIA), enzyme-linked immunosorbent assays (ELISAs), immunofluorescent assays, Western blotting and others.

### 3. Kits

In certain embodiments, one or more oligonucleotides disclosed herein are provided in a kit or on an array useful for detecting the presence of a predisposing or a protective polymorphism in a nucleic acid sample of an individual whose risk for a complement-related disease such as AMD and/or MPGNII is being assessed. A useful kit can contain oligonucleotide specific for particular alleles of interest as well as instructions for their use to determine risk for a complement-related disease such as AMD and/or MPGNII. In some cases, the oligonucleotides may be in a form suitable for use as a probe, for example fixed to an appropriate support membrane. In other cases, the oligonucleotides can be intended for use as amplification primers for amplifying regions of the loci encompassing the polymorphic sites, as such primers are useful in the preferred embodiment of the invention. Alternatively, useful kits can contain a set of primers comprising an allele-specific primer for the specific amplification of alleles. As yet another alternative, a useful kit can contain antibodies to a protein that is altered in expression levels, structure and/or sequence when a polymorphism of interest is present within an individual. Other optional components of the kits include additional reagents used in the genotyping methods as described herein. For example, a kit additionally can contain amplification or sequencing primers which can, but need not, be sequence-specific, enzymes, substrate nucleotides, reagents for labeling and/or detecting nucleic acid and/or appropriate buffers for amplification or hybridization reactions.

### 4. Arrays

Further disclosed is an array, a support with immobilized oligonucleotides useful for practicing the present method. A useful array can contain oligonucleotide probes specific for polymorphisms identified herein. The oligonucleotides can be immobilized on a substrate, e.g., a membrane or glass. The oligonucleotides can, but need not, be labeled. The array can comprise one or more oligonucleotides used to detect the presence of one or more SNPs provided herein. In some embodiments, the array can be a micro-array.

The array can include primers or probes to determine assay the presense or absence of at least two of the SNPs listed in Table I or II, sometimes at least three, at least four, at least five or at least six of the SNPs. In one embodiment, the array comprises probes or primers for detection of fewer than about 1000 different SNPs, often fewer than about 100 different SNPs, and sometimes fewer than about 50 different SNPs.

### VI. Nucleic Acids

Further disclosed are compositions comprising newly identified single nucleotide polymorphisms discovered in the FHR5 gene. The nucleic acids comprising variant FHR5 genes may be DNA or RNA and may be single or double stranded. In one embodiment, the variant allele of the FHR5 gene comprises the sequence TGCAGAAAAGGATGCGTGTGAACAGCAGGTAATTTTCTTCTGATTGATTCTATAT CTAGATGA (SEQ ID NO: 3). This sequence corresponds to the variant allele of MRD-3905, which has an "A" residue at the polymorphic site. In another embodiment, the variant allele of the FHR5 gene comprises the sequence GGGGAAAAGCAGTGTGGAAATTATTTAGGACTGTGTTCATTAATTTAAAGCAAG GCAAGTCAG (SEQ ID NO: 4). This sequence corresponds to the variant allele of MRD-3905, which has a "T" residue at the polymorphic site.

Further disclosed are vectors comprising the nucleic acid sequences encoding a variant FHR5 polypeptide (*e.g.,* a protective FHR5). The FHR5 polypeptide may be full length form or a truncated form. The variant FHR5 polypeptide may differ from normal or wild type FHR5 by a non-synonymous amino acid present at the polymorphic site.

Expression vectors for production of recombinant proteins and peptides are well known *(see* Ausubel et al., 2004, Current Protocols In Molecular Biology, Greene Publishing and Wiley-Interscience, New York). Such expression vectors include the nucleic acid sequence encoding the FRH5 polypeptide linked to regulatory elements, such a promoter, which drive transcription of the DNA and are adapted for expression in prokaryotic (e.g., E. coli) and eukaryotic (e.g., yeast, insect or mammalian cells) hosts. A variant FHR5 polypeptide can be expressed in an expression vector in which a variant FHR5 gene is operably linked to a promoter. Usually, the promoter is a eukaryotic promoter for expression in a mammalian cell. Usually, transcription regulatory sequences comprise a heterologous promoter and optionally an enhancer, which is recognized by the host cell. Commercially available expression vectors can be used. Expression vectors can include host-recognized replication systems, amplifiable genes, selectable markers, host sequences useful for insertion into the host genome, and the like.

Suitable host cells include bacteria such as E. coli, yeast, filamentous fungi, insect cells, and mammalian cells, which are typically immortalized, including mouse, hamster, human, and monkey cell lines, and derivatives thereof. Host cells may be able to process the variant FHR5 gene product to produce an appropriately processed, mature polypeptide. Such processing may include glycosylation, ubiquitination, disulfide bond formation, and the like.

Expression constructs containing a variant FHR5 gene are introduced into a host cell, depending upon the particular construction and the target host. Appropriate methods and host cells, both procarytic and eukaryotic, are well-known in the art. Recombinant full-length human FHR5 has been expressed for research purposes in Sf9 insect cells *(see* McRae et al., 2001, Human Factor H-related Protein 5 (FHR-5), J Biol. Chem. 276:6747-6754).

A variant FHR5 polypeptide may be isolated by conventional means of protein biochemistry and purification to obtain a substantially pure product. For general methods see Jacoby, Methods in Enzymology Volume 104, Academic Press, New York (1984); Scopes, Protein Purification, Principles and Practice, 2nd Edition, Springer-Verlag, New York (1987); and Deutscher (ed) Guide to Protein Purification, Methods in Enzymology, Vol. 182 (1990). Secreted proteins, like FHR5, can be isolated from the medium in which the host cell is cultured. If the variant FHR5 polypeptide is not secreted, it can be isolated from a cell lysate.

### VII. Antibodies

Further disclosed are FHR5-specific antibodies that may recognize a variant FHR5 polypeptide as described herein in which one or more non-synonymous single nucleotide polymorphisms (SNPS) are present in the FHR5 coding region. Further disclosed are antibodies that specifically recognize a variant FHR5 polypeptide described herein or fragments thereof, but not FHR5 polypeptides not having a variation at the polymorphic site.

The antibodies can be polyclonal or monoclonal, and are made according to standard protocols. Antibodies can be made by injecting a suitable animal with a variant FHR5 polypeptide, or fragment thereof, or synthetic peptide fragments thereof. Monoclonal antibodies are screened according to standard protocols (Koehler and Milstein 1975, Nature 256:495; Dower et al., WO 91/17271 and McCafferty et al., WO 92/01047; and Vaughan et al., 1996, Nature Biotechnology, 14: 309; and references provided below). In one embodiment, monoclonal antibodies are assayed for specific immunoreactivity with the FHR5 polypeptide, but not the corresponding wild-type FHR5 polypeptide, respectively. Methods to identify antibodies that specifically bind to a variant polypeptide, but not to the corresponding wild-type polypeptide, are well-known in the art. For methods, including antibody screening and subtraction methods; *see* Harlow & Lane, Antibodies, A Laboratory Manual, Cold Spring Harbor Press, New York (1988); Current Protocols in Immunology (J. E. Coligan et al., eds., 1999, including supplements through 2005); Goding, Monoclonal Antibodies, Principles and Practice (2d ed.) Academic Press, New York (1986); Burioni et al., 1998, "A new subtraction technique for molecular cloning of rare antiviral antibody specificities from phage display libraries" Res Virol. 149(5):327-30; Ames et al., 1994, Isolation of neutralizing anti-C5a monoclonal antibodies from a filamentous phage monovalent Fab display library. J Immunol. 152(9):4572-81; Shinohara et al., 2002, Isolation of monoclonal antibodies recognizing rare and dominant epitopes in plant vascular cell walls by phage display subtraction. J Immunol Methods 264(1-2):187-94. Immunization or screening can be directed against a full-length variant protein or, alternatively (and often more conveniently), against a peptide or polypeptide fragment comprising an epitope known to differ between the variant and wild-type forms. Particular variants include the P46S variant of FHR5. Monoclonal antibodies specific for variant FHR5 polypeptides (*i.e*., which do not bind wild-type proteins, or bind at a lower affinity) are useful in diagnostic assays for detection of the variant forms of CFHR5, or as an active ingredient in a pharmaceutical composition.

Further disclosed are recombinant polypeptides suitable for administration to patients including antibodies that are produced and tested in compliance with the Good Manufacturing Practice (GMP) requirements. For example, recombinant antibodies subject to FDA approval must be tested for potency and identity, be sterile, be free of extraneous material, and all ingredients in a product (*i.e.,* preservatives, diluents, adjuvants, and the like) must meet standards of purity, quality, and not be deleterious to the patient.

Further disclosed is a composition comprising an antibody that specifically recognizes a FHR5 polypeptide described herein (*e.g.,* a variant CFHR5 polypeptide) and a pharmaceutically acceptable excipient or carrier.

Further disclosed is a sterile container, e.g. vial, containing a therapeutically acceptable FHR5-specific antibody. In one embodiment it is a lyophilized preparation.

In a related aspect, the invention provides pharmaceutical preparations of human or humanized anti-FHR5 antibodies for administration to patients. Humanized antibodies have variable region framework residues substantially from a human antibody (termed an acceptor antibody) and complementarity determining regions substantially from a mouse-antibody, (referred to as the donor immunoglobulin). *See,* Peterson, 2005, Advances in monoclonal antibody technology: genetic engineering of mice, cells, and immunoglobulins, ILAR J. 46:314-9, Kashmiri et al., 2005, SDR grafting--a new approach to antibody humanization, Methods 356:25-34, Queen et al., Proc. Natl: Acad. Sci. USA 86:10029-10033 (1989), WO 90/07861, U.S. Pat. No. 5,693,762, U.S. Pat. No. 5,693,761, U.S. Pat. No. 5,585,089, U.S. Pat. No. 5,530,101, and Winter, U.S. Pat. No. 5,225,539. The constant region(s), if present, are also substantially or entirely from a human immunoglobulin. The human variable domains are usually chosen from human antibodies whose framework sequences exhibit a high degree of sequence identity with the murine variable region domains from which the CDRs were derived. The heavy and light chain variable region framework residues can be derived from the same or different human antibody sequences. The human antibody sequences can be the sequences of naturally occurring human antibodies or can be consensus sequences of several human antibodies. *See* Carter et al., WO 92/22653. Certain amino acids from the human variable region framework residues are selected for substitution based on their possible influence on CDR conformation and/or binding to antigen. Investigation of such possible influences is by modeling, examination of the characteristics of the amino acids at particular locations, or empirical observation of the effects of substitution or mutagenesis of particular amino acids.

For example, when an amino acid differs between a murine variable region framework residue and a selected human variable region framework residue, the human framework amino acid should usually be substituted by the equivalent framework amino acid from the mouse antibody when it is reasonably expected that the amino acid: (1) noncovalently binds antigen directly, (2) is adjacent to a CDR region, (3) otherwise interacts with a CDR region (e.g. is within about 6 A of a CDR region), or (4) participates in the VL-VH interface.

Other candidates for substitution are acceptor human framework amino acids that are unusual for a human immunoglobulin at that position. These amino acids can be substituted with amino acids from the equivalent position of the mouse donor antibody or from the equivalent positions of more typical human immunoglobulins. Other candidates for substitution are acceptor human framework amino acids that are unusual for a human immunoglobulin at that position. The variable region frameworks of humanized immunoglobulins usually show at least 85% sequence identity to a human variable region framework sequence or consensus of such sequences.

### VIII. Therapeutic Methods

Further disclosed is a method for treating or preventing AMD or MPGNII, comprising prophylactically or therapeutically treating an individual identified as having a genetic profile in the regulation of the complement activation (RCA) locus of chromosome one extending from CFH through F13B indicative of increased risk of development or progression of AMD or MPGNII, wherein the genetic profile comprises one or more single nucleotide polymorphisms selected from Table I, Table IA, or Table II.

An individual with a genetic profile indicative of AMD and/or MPGNII can be treated by administering a composition comprising a human Complement Factor H polypeptide to the individual. In one embodiment, the Factor H polypeptide is encoded by a Factor H protective haplotype. A protective Factor H haplotype can encode an isoleucine residue at amino acid position 62 and/or an amino acid other than a histidine at amino acid position 402. For example, a Factor H polypeptide can comprise an isoleucine residue at amino acid position 62, a tyrosine residue at amino acid position 402, and/or an arginine residue at amino acid position 1210. Exemplary Factor H protective haplotypes include the H2 haplotype or the H4 haplotype (*see* U.S. Patent Publication 2007/0020647). Alternatively, the Factor H polypeptide may be encoded by a Factor H neutral haplotype. A neutral haplotype encodes an amino acid other than an isoleucine at amino acid position 62 and an amino acid other than a histidine at amino acid position 402. Exemplary Factor H neutral haplotypes include the H3 haplotype or the H5 haplotype (*see* U.S. Patent Publication 2007/0020647).

A therapeutic Factor H polypeptide may be a recombinant protein or it may be purified from blood. A Factor H polypeptide may be administered to the eye by intraocular injection or systemically.

Alternatively, or in addition, an individual with a genetic profile indicative of elevated risk of AMD could be treated by inhibiting the expression or activity of HTRA1. As one example, HTRA1 can be inhibited by administering an antibody or other protein *(e.g.* an antibody variable domain, an addressable fibronectin protein, *etc*.) that binds HTRA1. Alternatively, HTRA1 can be inhibited by administering a small molecule that interferes with HTRA1 activity (e.g. an inhibitor of the protease activity of HTRA1) or a nucleic acid inhibiting HTRA1 expression or activity, such as an inhibitory RNA *(e.g.* a short interfering RNA, a short hairpin RNA, or a microRNA), a nucleic acid encoding an inhibitory RNA, an antisense nucleic acid, or an aptamer that binds HTRA1. See, for example, International Publication No. WO 2008/013893. An inhibitor for HTRA1 activity, NVP-LBG976, is available from Novartis, Basel *(see* also, Grau S, PNAS, (2005) 102: 6021-6026). Antibodies reactive to HTRA1 are commercially available (for example from Imgenex) and are also described in, for example, PCT application No. WO 00/08134.

Alternatively, or in addition, the method of treating or preventing AMD in an individual includes prophylactically or therapeutically treating the individual by inhibiting Factor B and/or C2 in the individual. Factor B can be inhibited, for example, by administering an antibody or other protein *(e.g.,* an antibody variable domain, an addressable fibronectin protein, *etc*.) that binds Factor B. Alternatively, Factor B can be inhibited by administering a nucleic acid inhibiting Factor B expression or activity, such as an inhibitory RNA, a nucleic acid encoding an inhibitory RNA, an antisense nucleic acid, or an aptamer, or by administering a small molecule that interferes with Factor B activity (e.g., an inhibitor of the protease activity of Factor B). C2 can be inhibited, for example, by administering an antibody or other protein (*e.g.,* an antibody variable domain, an addressable fibronectin protein, *etc*.) that binds C2. Alternatively, C2 can be inhibited by administering a nucleic acid inhibiting C2 expression or activity, such as an inhibitory RNA, a nucleic acid encoding an inhibitory RNA, an antisense nucleic acid, or an aptamer, or by administering a small molecule that interferes with C2 activity (*e*.*g*., an inhibitor of the protease activity of C2).

In another embodiment, an individual with a genetic profile indicative of AMD (*i.e.,* the individual's genetic profile comprises one or more single nucleotide polymorphisms selected from Table I, Table IA or Table II) can be treated by administering a composition comprising a C3 convertase inhibitor, *e.g.,* compstatin (*See e.g.* PCT publication WO 2007/076437). optionally in combination with a therapeutic factor H polypeptide. In another embodiment, an individual with a genetic profile indicative of AMD and who is diagnosed with AMD may be treated with an angiogenic inhibitor such as anecortave acetate (RETAANE®, Alcon), an anti-VEGF inhibitor such as pegaptanib (Macugen®, Eyetech Pharmaceuticals and Pfizer, Inc.) and ranibizumab (Lucentis®, Genentech), and/or verteporfin (Visudyne®, QLT, Inc./Novartis).

### IX. Authorization of Treatment or Payment for Treatment

Further disclosed is a healthcare method comprising paying for, authorizing payment for or authorizing the practice of the method of screening for susceptibility to developing or for predicting the course of progression of AMD and/or MPGNII in an individual, comprising screening for the presence or absence of a genetic profile in the regulation of the complement activation (RCA) locus of chromosome one extending from FHR1 through F13B, wherein the genetic profile comprises one or more single nucleotide polymorphisms selected from Table I, Table IA, or II.

A third party, *e.g*., a hospital, clinic, a government entity, reimbursing party, insurance company (e.g., a health insurance company), HMO, third-party payor, or other entity which pays for, or reimburses medical expenses may authorize treatment, authorize payment for treatment, or authorize reimbursement of the costs of treatment. For example, disclosed is a healthcare method that includes authorizing the administration of, or authorizing payment or reimbursement for the administration of, a diagnostic assay for determining an individual's susceptibility for developing or for predicting the course of progression of AMD and/or MPGNII as disclosed herein. For example, the healthcare method can include authorizing the administration of, or authorizing payment or reimbursement for the administration of, a diagnostic assay to determine an individual's susceptibility for development or progression of AMD and/or MPGNII comprising screening for the presence or absence of a genetic profile in the RCA locus of chromosome one extending from CFH to F13B, wherein the genetic profile comprises one or more SNPs selected from Table I, Table IA, or II.

### X. Complement-related diseases

The polymorphisms provided herein have a statistically significant association with one or more disorders that involve dysfunction of the complement system. In certain embodiments, an individual may have a genetic predisposition based on their genetic profile to developing more than one disorder associated with dysregulation of the complement system. For example, said individual's genetic profile may comprise one or more polymorphism shown in Table I, Table IA and/or II, wherein the genetic profile is informative of AMD and another disease characterized by dysregulation of the complement system. Accordingly, disclosed is the use of these polymorphisms for assessing an individual's risk for any complement-related disease or condition, including but not limited to AMD and/or MPGNII. Other complement-related diseases include Barraquer-Simons Syndrome, asthma, lupus erythematosus, glomerulonephritis, various forms of arthritis including rheumatoid arthritis, autoimmune heart disease, multiple sclerosis, inflammatory bowel disease, Celiac disease, diabetes mellitus type 1, Sjögren's syndrome, and ischemia-reperfusion injuries. The complement system is also becoming increasingly implicated in diseases of the central nervous system such as Alzheimer's disease, and other neurodegenerative conditions. Applicant suspects that many patients may die of disease caused in part by dysfunction of the complement cascade well before any symptoms of AMD appear. Accordingly, the invention disclosed herein may well be found to be useful in early diagnosis and risk assessment of other disease, enabling opportunistic therapeutic or prophylactic intervention delaying the onset or development of symptoms of such disease.

The examples of the present invention presented below are provided only for illustrative purposes and not to limit the scope of the invention. Numerous embodiments of the invention within the scope of the claims that follow the examples will be apparent to those of ordinary skill in the art from reading the foregoing text and following examples.

### EXAMPLES

Additional sub-analyses were performed to support data derived from analyses described above in Tables I-II. These include:
Sub-analysis 1: One preliminary sub-analysis was performed on a subset of 2,876 SNPs using samples from 590 AMD cases and 375 controls. It was determined that this sample provided adequate power (>80%) for detecting an association between the selected markers and AMD (for a relative risk of 1.7, a sample size of 500 per group was required, and for a relative risk of 1.5, the sample size was calculated to be 700 per group).

The raw data were prepared for analysis in the following manner: 1) SNPs with more than 5% failed calls were deleted (45 total SNPs); 2) SNPs with no allelic variation were deleted (354 alleles); 3) subjects with more than 5% missing genotypes were deleted (11 subjects); and 4) the 2,876 remaining SNPs were assessed for LD, and only one SNP was retained for each pair with r2 > 0.90 (631 SNPs dropped, leaving 2245 SNPs for analysis). Genotype associations were assessed using a statistical software program (*i.e*., SAS ® PROC CASECONTROL) and the results were sorted both by genotype p-value and by allelic p-value. For 2,245 SNPs, the Bonferroni - corrected alpha level for significance is 0.00002227. Seventeen markers passed this test. HWE was assessed for each of the 17 selected markers, both with all data combined and by group.

AMD-associated SNPs were further analyzed to determine q-values. Of 2245 SNPs analyzed, 74 SNPs were shown to be associated with AMD at a q-value less than 0.50. The first section of SNPs represent loci that passed the Bonferroni condition. The second section of SNPs were those that didn't make the Bonferroni cut-off, but had q-values less than 0.20; the third section of SNPs had q-values greater than 0.20, but less than 0.50. 16 AMD-associated SNPs, located in the CFH, LOC387715, FHR4, FHR5, PRSS11, PLEKHA1 and FHR2 genes passed the Bonferroni level of adjustment. These results confirm the published associations of the CFH and LOC387715, PLEKHA1 and PRSS11 genes with AMD. 14 additional SNPs located within the FHR5, FHR2, CFH, PRSS11, FHR1, SPOCK3, PLEKHA1, C2, FBN2, TLR3 and SPOCK loci were significantly associated with AMD; these SNPs didn't pass the Bonferroni cut-off, but had q-values less than 0.20 (after adjusting for false discovery rate). In addition, another 27 SNPs were significantly associated with AMD (p<0.05) at q-values between 0.20 and 0.50.

These data confirm existing gene associations in the literature. They also provide evidence that other complement-associated genes (*e.g.,* FHR1, FHR2, FHR4, FHR5) may not be in linkage disequilibrium (LD) with CFH and, if replicated in additional cohorts, may be independently associated with AMD. It is also noted that FHR1, FHR2 and FHR4 are in the same LD bin and further genotyping will be required to identify the gene(s) within this group that drive the detected association with AMD.

Sub-analysis 2: Another sub-analysis was performed on a subset comprised of 516 AMD cases and 298 controls using criteria as described above. A total of 3,266 SNPs in 352 genes from these regions were tested. High significance was detected for previously established AMD-associated genes, as well as for several novel AMD genes. SNPs exhibiting p values <0.01 and difference in allele frequencies >10%, and >5%, are depicted in Table I.

Sub-analysis 3: Another sub-analysis was performed comparing 499 AMD cases to 293 controls; data were assessed for Hardy-Weinberg association, analyzed by Chi Square. Using a cutoff of p<0.005, 40 SNPs were significantly associated with AMD; these included SNPs within genes shown previously to be associated with AMD (CFH/ENSG00000000971, CFHR1, CFHR2, CFHR4, CFHR5, F13B, PLEKHA1, LOC387715 and PRSS11/HTRA1), as well as additional strong associations with CCL28 and ADAM12. The same samples were analyzed also by conditioning on the CFH Y402H SNP to determine how much association remained after accounting for this strongly associated SNP using a Cochran-Armitage Chi Square test for association within a bin and a Mantel-Haenszel test for comparing bins. The significance of association for most markers in the CFH region drops or disappears after stratification for Y402H, but this SNP has no effect on the PLEKHA1, LOC387715, PRSS11/HTRA1, CCL28 or ADAM12. Similarly LOC3877156 SNP rs3750847 has no effect on association on chromosome 1 SNPs, although association with chromosome 10-associated SNPs disappears except for ADAM12. Thus, the ADAM12 association is not in LD with the previously established AMD locus on chromosome 10 (PLEKHA1, LOC387715, and PRSS11/HTRA1 genes). The ADAM12 signal appears to be coming from association with the over 84 group.

Sub-analysis 4: When the control group (N=293) is compared to the MPGNII cohort (N=18), SNPs associated with the CFH gene comes up strongly, as previously published (Table II). The signal decreases when the data are conditioned on the Y402H SNP; the remaining signal on chromosome 1 appears to be associated with a deletion of the FHR1 and FHR3 genes (the signal decreases when one stratifies the data by groups that roughly reflect copy number of the deletion), as previously published. New associations with MPGNII include SNPs within the CFH, F13B, FHR1, FHR2, FHR4 and FHR5.

**Table I: Polymorphisms Associated with AMD**

| Gene | SNP | Allele 1/ Allele 2 | Allele Frequencies (percentages): Control Population | | | | | Allele Frequencies (percentages): Disease Population | | | | | Genotype-Likelihood Ratio (3 categories) | Frequencies Chi Square (both collapsed- 2 categories) |
|---|---|---|---|---|---|---|---|---|---|---|---|---|---|---|
| | | | Homozygotes | | Heterozygotes | Allele 1 Overall | Allele 2 Overall | Homozygotes | | Heterozygotes | Allele 1 Overall | Allele 2 Overall | | |
| | | | Allele 1 | Allele 2 | | | | Allele 1 | Allele 2 | | | | | |
| F13B | rs5997 | A/G | 1 | 77.9 | 21 | 11.6 | 88.4 | 0.4 | 90.1 | 9.5 | 5.2 | 94.8 | 2.48E-05 | 3.37E-06 |
| F13B | rs6428380 | A/G | 1 | 78.4 | 20.6 | 11.3 | 88.7 | 0.4 | 90.1 | 9.5 | 5.2 | 94.8 | 4.11E-05 | 5.81E-06 |
| F13B | rs1794006 | C/T | 78.4 | 1 | 20.6 | 88.7 | 11.3 | 89.9 | 0.4 | 9.7 | 94.7 | 5.3 | 6.13E-05 | 8.87E-06 |
| F13B | rs10801586 | C/T | 69.6 | 2 | 28.4 | 83.8 | 16.2 | 82.2 | 1.4 | 16.4 | 90.4 | 9.6 | 2.43E-04 | 8.70E-05 |
| FHR1 | rs12027476 | C/G | 0 | 63.6 | 36.4 | 18.2 | 81.8 | 0.0 | 78.2 | 21.8 | 10.9 | 89.1 | 1.24E-05 | 4.99E-05 |
| FHR1 | rs436719 | A/C | 46.6 | 0 | 53.4 | 73.3 | 26.7 | 58.8 | 0.0 | 41.2 | 79.4 | 20.6 | 8.32E-04 | 5.04E-03 |
| FHR2 | rs12066959 | A/G | 5.5 | 58.7 | 35.8 | 23.4 | 76.6 | 2.0 | 75.0 | 23.0 | 13.5 | 86.5 | 4.83E-06 | 4.38E-07 |
| FHR2 | rs3828032 | A/G | 8.2 | 46.3 | 45.6 | 31.0 | 69.0 | 5.0 | 62.7 | 32.3 | 21.1 | 78.9 | 3.29E-05 | 1.16E-05 |
| FHR2 | rs6674522 | C/G | 1.4 | 76.7 | 22 | 12.3 | 87.7 | 0.4 | 87.9 | 11.7 | 6.2 | 93.8 | 1.79E-04 | 2.40E-05 |
| FHR2 | rs432366 | C/G | 0 | 47 | 53 | 26.5 | 73.5 | 0.0 | 58.8 | 41.2 | 20.6 | 79.4 | 1.15E-03 | 6.34E-03 |
| FHR4 | rs1409153 | A/G | 36.1 | 14.9 | 49 | 60.6 | 39.4 | 17.0 | 36.8 | 46.1 | 40.1 | 59.9 | 3.25E-14 | 1.93E-15 |
| FHR5 | MRD_3905 | A/G | 3 | 57.8 | 39.2 | 22.6 | 77.4 | 3.4 | 68.9 | 27.7 | 17.2 | 82.8 | 3.74E-03 | 8.03E-03 |
| FHR5 | MRD 3906 | C/T | 57.8 | 3.7 | 38.5 | 77.0 | 23.0 | 68.5 | 3.4 | 28.1 | 82.6 | 17.4 | 8.16E-03 | 6.81E-03 |

**Table IA: Additional Polymorphism Associated with AMD**

| Gene | SNP | Allele 1/ Allele 2 | Allele Frequencies (percentages): Control Population | | | | | Allele Frequencies (percentages): Disease Population | | | | | Genotype-Likelihood Ratio (3 categories) | Frequencies Chi Square (both collapsed- 2 categories) |
|---|---|---|---|---|---|---|---|---|---|---|---|---|---|---|
| | | | Homozygotes | | Heterozygotes | Allele 1 Overall | Allele 2 Overall | Homozygotes | | Heterozygotes | Allele 1 Overall | Allele 2 Overall | | |
| | | | Allele 1 | Allele 2 | | | | Allele 1 | Allele 2 | | | | | |
| FHR5 | rs10922153 | G/T | 23.6 | 25.7 | 50.7 | 49.0 | 51.0 | 44.6 | 9.5 | 45.9 | 67.5 | 32.5 | 1.38E-12 | 2.27E-13 |

**Table II: Polymorphisms Associated with MPGNII**

| Gene | SNP | Allele 1/ Allele 2 | Allele Frequencies (percentages): Control Population | | | | | Allele Frequencies (percentages): Disease Population | | | | | Genotype-Likelihood Ratio (3 categories) | Freq.Chi Square (both collapsed-2 categories) | Freq.Fishers Exact (both collapsed-2 categories) |
|---|---|---|---|---|---|---|---|---|---|---|---|---|---|---|---|
| | | | Homozygotes | | Hetero-zygotes | Allele 1 Overall Freq. | Allele 2 Overall Freq. | Homozygotes | | Hetero-zygotes | Allele 1 Overall Freq. | Allele 2 Overall Freq. | | | |
| | | | Allele 1 | Allele 2 | | | | Allele 1 | Allele 2 | | | | | | |
| CFH | rs3753395 | A/T | 34.8 | 17.6 | 47.6 | 58.6 | 41.4 | 84.2 | 0.0 | 15.8 | 92.1 | 7.9 | 3.68E-05 | 4.20E-05 | 1.10E-05 |
| CFH | rs1410996 | C/T | 34.8 | 17.6 | 47.6 | 58.6 | 41.4 | 84.2 | 0.0 | 15.8 | 92.1 | 7.9 | 3.68E-05 | 4.20E-05 | 1.10E-05 |
| CFH | rs1329421 | A/T | 39.5 | 15.2 | 45.3 | 62.2 | 37.8 | 10.5 | 42.1 | 47.4 | 34.2 | 65.8 | 3.99E-03 | 6.35E-04 | 9.42E-04 |
| CFH | rs10801554 | C/T | 15.2 | 39.5 | 45.3 | 37.8 | 62.2 | 42.1 | 10.5 | 47.4 | 65.8 | 34.2 | 3.99E-03 | 6.35E-04 | 9.42E-04 |
| CFH | rs12124794 | A/T | 64.7 | 5.8 | 29.5 | 79.5 | 20.5 | 94.7 | 0.0 | 5.3 | 97.4 | 2.6 | 7.46E-03 | 6.94E-03 | 4.76E-03 |
| CFH | rs393955 | G/T | 17.9 | 33.1 | 49.0 | 42.4 | 57.6 | 47.4 | 10.5 | 42.1 | 68.4 | 31.6 | 7.53E-03 | 1.73E-03 | 2.15E-03 |
| CFH | rs403846 | A/G | 17.9 | 33.1 | 49.0 | 42.4 | 57.6 | 47.4 | 10.5 | 42.1 | 68.4 | 31.6 | 7.53E-03 | 1.73E-03 | 2.15E-03 |
| CFH | rs2284664 | A/G | 5.4 | 65.2 | 29.4 | 20.1 | 79.9 | 0.0 | 94.7 | 5.3 | 2.6 | 97.4 | 8.38E-03 | 7.85E-03 | 4.67E-03 |
| CFH | rs12144939 | G/T | 62.2 | 4.7 | 33.1 | 78.7 | 21.3 | 89.5 | 0.0 | 10.5 | 94.7 | 5.3 | 2.44E-02 | 1.73E-02 | 1.26E-02 |
| F13B | rs2990510 | G/T | 8.4 | 45.6 | 45.9 | 31.4 | 68.6 | 26.3 | 21.1 | 52.6 | 52.6 | 47.4 | 2.58E-02 | 6.89E-03 | 1.14E-02 |
| FHR1 | rs12027476 | C/G | 0.0 | 63.6 | 36.4 | 18.2 | 81.8 | 0.0 | 89.5 | 10.5 | 5.3 | 94.7 | 1.21E-02 | 4.17E-02 | 4.49E-02 |
| FHR2 | rs12066959 | A/G | 5.5 | 58.7 | 35.8 | 23.4 | 76.6 | 0.0 | 89.5 | 10.5 | 5.3 | 94.7 | 1.13E-02 | 9.30E-03 | 7.72E-03 |
| FHR2 | rs4085749 | C/T | 59.0 | 5.4 | 35.6 | 76.8 | 23.2 | 89.5 | 0.0 | 10.5 | 94.7 | 5.3 | 1.20E-02 | 9.75E-03 | 7.70E-03 |
| FHR4 | rs1409153 | A/G | 36.1 | 14.9 | 49.0 | 60.6 | 39.4 | 10.5 | 47.4 | 42.1 | 31.6 | 68.4 | 1.93E-03 | 4.16E-04 | 5.54E-04 |

**Table III A. AMD Control Population Cases**

| Gene | SNP | Allele 1/ Allele 2 | Control Undeter. Freq. | Control N | Allele Frequencies: Control Population | | | Allele Frequencies (percentages): Control Population | | | | |
|---|---|---|---|---|---|---|---|---|---|---|---|---|
| | | | | | Homozygotes | | Heterozygotes | Homozygotes | | Heterozygotes | Allele 1 Overall | Allele 2 Overall |
| | | | | | Allele 1 | Allele 2 | | Allele 1 | Allele 2 | | | |
| F13B | rs5997 | A/G | 6 | 290 | 3 | 226 | 61 | 1 | 77.9 | 21 | 11.6 | 88.4 |
| F13B | rs6428380 | A/G | 0 | 296 | 3 | 232 | 61 | 1 | 78.4 | 20.6 | 11.3 | 88.7 |
| F13B | rs1794006 | C/T | 0 | 296 | 232 | 3 | 61 | 78.4 | 1 | 20.6 | 88.7 | 11.3 |
| F13B | rs10801586 | C/T | 0 | 296 | 206 | 6 | 84 | 69.6 | 2 | 28.4 | 83.8 | 16.2 |
| FHR1 | rs12027476 | C/G | 13 | 283 | 0 | 180 | 103 | 0 | 63.6 | 36.4 | 18.2 | 81.8 |
| FHR1 | rs436719 | A/C | 0 | 296 | 138 | 0 | 158 | 46.6 | 0 | 53.4 | 73.3 | 26.7 |
| FHR2 | rs12066959 | A/G | 3 | 293 | 16 | 172 | 105 | 5.5 | 58.7 | 35.8 | 23.4 | 76.6 |
| FHR2 | rs3828032 | A/G | 2 | 294 | 24 | 136 | 134 | 8.2 | 46.3 | 45.6 | 31.0 | 69.0 |
| FHR2 | rs6674522 | C/G | 0 | 296 | 4 | 227 | 65 | 1.4 | 76.7 | 22 | 12.3 | 87.7 |
| FHR2 | rs432366 | C/G | 0 | 296 | 0 | 139 | 157 | 0 | 47 | 53 | 26.5 | 73.5 |
| FHR4 | rs1409153 | A/G | 0 | 296 | 107 | 44 | 145 | 36.1 | 14.9 | 49 | 60.6 | 39.4 |
| FHR5 | MRD_3905 | A/G | 0 | 296 | 9 | 171 | 116 | 3 | 57.8 | 39.2 | 22.6 | 77.4 |
| FHR5 | MRD_3906 | C/T | 0 | 296 | 171 | 11 | 114 | 57.8 | 3.7 | 38.5 | 77.0 | 23.0 |
| FHR5 | rs10922153 | G/T | 0 | 296 | 70 | 76 | 150 | 23.6 | 25.7 | 50.7 | 49.0 | 51.0 |

**Table III B. AMD Disease Population Cases**

| Gene | SNP | Allele 1/ Allele 2 | Disease Undeter. Freq. | Disease N | Allele Frequencies: Disease Population | | | Allele Frequencies (percentages): Disease Population | | | | |
|---|---|---|---|---|---|---|---|---|---|---|---|---|
| | | | | | Homozygotes | | Heterozygotes | Homozygotes | | Heterozygotes | Allele 1 Overall | Allele 2 Overall |
| | | | | | Allele 1 | Allele 2 | | Allele 1 | Allele 2 | | | |
| F13B | rs5997 | A/G | 2 | 503 | 2 | 453 | 48 | 0.4 | 90.1 | 9.5 | 5.2 | 94.8 |
| F13B | rs6428380 | A/G | 1 | 504 | 2 | 454 | 48 | 0.4 | 90.1 | 9.5 | 5.2 | 94.8 |
| F13B | rs1794006 | C/T | 1 | 504 | 453 | 2 | 49 | 89.9 | 0.4 | 9.7 | 94.7 | 5.3 |
| F13B | rs10801586 | C/T | 0 | 505 | 415 | 7 | 83 | 82.2 | 1.4 | 16.4 | 90.4 | 9.6 |
| FHR1 | rs12027476 | C/G | 9 | 496 | 0 | 388 | 108 | 0.0 | 78.2 | 21.8 | 10.9 | 89.1 |
| FHR1 | rs436719 | A/C | 0 | 505 | 297 | 0 | 208 | 58.8 | 0.0 | 41.2 | 79.4 | 20.6 |
| FHR2 | rs12066959 | A/G | 1 | 504 | 10 | 378 | 116 | 2.0 | 75.0 | 23.0 | 13.5 | 86.5 |
| FHR2 | rs3828032 | A/G | 1 | 504 | 25 | 316 | 163 | 5.0 | 62.7 | 32.3 | 21.1 | 78.9 |
| FHR2 | rs6674522 | C/G | 0 | 505 | 2 | 444 | 59 | 0.4 | 87.9 | 11.7 | 6.2 | 93.8 |
| FHR2 | rs432366 | C/G | 0 | 505 | 0 | 297 | 208 | 0.0 | 58.8 | 41.2 | 20.6 | 79.4 |
| FHR4 | rs1409153 | A/G | 0 | 505 | 86 | 186 | 233 | 17.0 | 36.8 | 46.1 | 40.1 | 59.9 |
| FHR5 | MRD_3905 | A/G | 0 | 505 | 17 | 348 | 140 | 3.4 | 68.9 | 27.7 | 17.2 | 82.8 |
| FHR5 | MRD_3906 | C/T | 0 | 505 | 346 | 17 | 142 | 68.5 | 3.4 | 28.1 | 82.6 | 17.4 |
| FHR5 | rs10922153 | G/T | 0 | 505 | 225 | 48 | 232 | 44.6 | 9.5 | 45.9 | 67.5 | 32.5 |

**Table III C. Differences in Allele Frequencies between AMD Control and Disease Populations**

| Gene | SNP | Allele 1/ Allele 2 | Difference in Percentage Allele Freqeuency (Allele 1) | Difference in Percentage (Hetero-Both) | Difference in Percentage Allele Freqeuency (Allele 2) | Difference in Percentage (Undeterrmined) |
|---|---|---|---|---|---|---|
| F13B | rs5997 | A/G | 1 | 21 | 77.9 | 1.6 |
| F13B | rs6428380 | A/G | 1 | 20.6 | 78.4 | 0.2 |
| F13B | rs1794006 | C/T | 78.4 | 20.6 | 1 | 0.2 |
| F13B | rs10801586 | C/T | 69.6 | 28.4 | 2 | 0.0 |
| FHR1 | rs12027476 | C/G | 0 | 36.4 | 63.6 | 2.6 |
| FHR1 | rs436719 | A/C | 46.6 | 53.4 | 0 | 0.0 |
| FHR2 | rs12066959 | A/G | 5.5 | 35.8 | 58.7 | 0.8 |
| FHR2 | rs3828032 | A/G | 8.2 | 45.6 | 46.3 | 0.5 |
| FHR2 | rs6674522 | C/G | 1.4 | 22 | 76.7 | 0.0 |
| FHR2 | rs432366 | C/G | 0 | 53 | 47 | 0.0 |
| FHR4 | rs1409153 | A/G | 36.1 | 49 | 14.9 | 0.0 |
| FHR5 | MRD_3905 | A/G | 3 | 39.2 | 57.8 | 0.0 |
| FHR5 | MRD_3906 | C/T | 57.8 | 38.5 | 3.7 | 0.0 |
| FHR5 | rs10922153 | G/T | 23.6 | 50.7 | 25.7 | 0.0 |

**Table IV A. MPGN II Control Population Cases**

| Gene | SNP | Allele 1/ Allele 2 | Control Undeter. Freq. | Control N | Allele Frequencies: Control Population | | | Allele Frequencies (percentages): Control Population | | | | |
|---|---|---|---|---|---|---|---|---|---|---|---|---|
| | | | | | Homozygotes | | Heterozygotes | Homozygotes | | Heterozygotes | Allele 1 Overall | Allele 2 Overall |
| | | | | | Allele 1 | Allele 2 | | Allele 1 | Allele 2 | | | |
| CFH | rs3753395 | A/T | 0 | 296 | 103 | 52 | 141 | 34.8 | 17.6 | 47.6 | 58.6 | 41.4 |
| CFH | rs1410996 | C/T | 0 | 296 | 103 | 52 | 141 | 34.8 | 17.6 | 47.6 | 58.6 | 41.4 |
| CFH | rs1329421 | A/T | 0 | 296 | 117 | 45 | 134 | 39.5 | 15.2 | 45.3 | 62.2 | 37.8 |
| CFH | rs10801554 | C/T | 0 | 296 | 45 | 117 | 134 | 15.2 | 39.5 | 45.3 | 37.8 | 62.2 |
| CFH | rs12124794 | A/T | 1 | 295 | 191 | 17 | 87 | 64.7 | 5.8 | 29.5 | 79.5 | 20.5 |
| CFH | rs393955 | G/T | 0 | 296 | 53 | 98 | 145 | 17.9 | 33.1 | 49.0 | 42.4 | 57.6 |
| CFH | rs403846 | A/G | 0 | 296 | 53 | 98 | 145 | 17.9 | 33.1 | 49.0 | 42.4 | 57.6 |
| CFH | rs2284664 | A/G | 0 | 296 | 16 | 193 | 87 | 5.4 | 65.2 | 29.4 | 20.1 | 79.9 |
| CFH | rs12144939 | G/T | 0 | 296 | 184 | 14 | 98 | 62.2 | 4.7 | 33.1 | 78.7 | 21.3 |
| F13B | rs2990510 | G/T | 0 | 296 | 25 | 135 | 136 | 8.4 | 45.6 | 45.9 | 31.4 | 68.6 |
| FHR1 | rs12027476 | C/G | 13 | 283 | 0 | 180 | 103 | 0.0 | 63.6 | 36.4 | 18.2 | 81.8 |
| FHR2 | rs12066959 | A/G | 3 | 293 | 16 | 172 | 105 | 5.5 | 58.7 | 35.8 | 23.4 | 76.6 |
| FHR2 | rs4085749 | C/T | 1 | 295 | 174 | 16 | 105 | 59.0 | 5.4 | 35.6 | 76.8 | 23.2 |
| FHR4 | rs1409153 | A/G | 0 | 296 | 107 | 44 | 145 | 36.1 | 14.9 | 49.0 | 60.6 | 39.4 |

**Table IV B. MPGN II Disease Population Cases**

| Gene | SNP | Allele 1/ Allele 2 | Disease Undeter. Freq. | Disease N | Allele Frequencies: Disease Population | | | Allele Frequencies (percentages): Disease Population | | | | |
|---|---|---|---|---|---|---|---|---|---|---|---|---|
| | | | | | Homozygotes | | Heterozygotes | Homozygotes | | Heterozygotes | Allele 1 Overall | Allele 2 Overall |
| | | | | | Allele 1 | Allele 2 | | Allele 1 | Allele 2 | | | |
| CFH | rs3753395 | A/T | 0 | 19 | 16 | 0 | 3 | 84.2 | 0.0 | 15.8 | 92.1 | 7.9 |
| CFH | rs1410996 | C/T | 0 | 19 | 16 | 0 | 3 | 84.2 | 0.0 | 15.8 | 92.1 | 7.9 |
| CFH | rs1329421 | A/T | 0 | 19 | 2 | 8 | 9 | 10.5 | 42.1 | 47.4 | 34.2 | 65.8 |
| CFH | rs10801554 | G/T | 0 | 19 | 8 | 2 | 9 | 42.1 | 10.5 | 47.4 | 65.8 | 34.2 |
| CFH | rs12124794 | A/T | 0 | 19 | 18 | 0 | 1 | 94.7 | 0.0 | 5.3 | 97.4 | 2.6 |
| CFH | rs393955 | G/T | 0 | 19 | 9 | 2 | 8 | 47.4 | 10.5 | 42.1 | 68.4 | 31.6 |
| CFH | rs403846 | A/G | 0 | 19 | 9 | 2 | 8 | 47.4 | 10.5 | 42.1 | 68.4 | 31.6 |
| CFH | rs2284664 | A/G | 0 | 19 | 0 | 18 | 1 | 0.0 | 94.7 | 5.3 | 2.6 | 97.4 |
| CFH | rs12144939 | G/T | 0 | 19 | 17 | 0 | 2 | 89.5 | 0.0 | 10.5 | 94.7 | 5.3 |
| F13B | rs2990510 | G/T | 0 | 19 | 5 | 4 | 10 | 26.3 | 21.1 | 52.6 | 52.6 | 47.4 |
| FHR1 | rs12027476 | C/G | 0 | 19 | 0 | 17 | 2 | 0.0 | 89.5 | 10.5 | 5.3 | 94.7 |
| FHR2 | rs12066959 | A/G | 0 | 19 | 0 | 17 | 2 | 0.0 | 89.5 | 10.5 | 5.3 | 94.7 |
| FHR2 | rs4085749 | C/T | 0 | 19 | 17 | 0 | 2 | 89.5 | 0.0 | 10.5 | 94.7 | 5.3 |
| FHR4 | rs1409153 | A/G | 0 | 19 | 2 | 9 | 8 | 10.5 | 47.4 | 42.1 | 31.6 | 68.4 |

**Table IV C. Differences in Allele Frequencies between MPGNII Control and Disease Populations**

| Gene | SNP | Allele 1/ Allele 2 | Difference in Percentage Allele Freqeuency (Allele 1) | Difference in Percentage (Hetero-Both) | Difference in Percentage Allele Freqeuency (Allele 2) | Difference in Percentage (Undeterrmined) |
|---|---|---|---|---|---|---|
| CFH | rs3753395 | A/T | 49.4 | 31.8 | 17.6 | 0 |
| CFH | rs1410996 | C/T | 49.4 | 31.8 | 17.6 | 0 |
| CFH | rs1329421 | A/T | 29 | 2.1 | 26.9 | 0 |
| CFH | rs10801554 | C/T | 26.9 | 2.1 | 29 | 0 |
| CFH | rs12124794 | A/T | 30 | 24.2 | 5.8 | 0.337838 |
| CFH | rs393955 | G/T | 29.5 | 6.9 | 22.6 | 0 |
| CFH | rs403846 | A/G | 29.5 | 6.9 | 22.6 | 0 |
| CFH | rs2284664 | A/G | 5.4 | 24.1 | 29.5 | 0 |
| CFH | rs12144939 | G/T | 27.3 | 22.6 | 4.7 | 0 |
| F13B | rs2990510 | G/T | 17.9 | 6.7 | 24.5 | 0 |
| FHR1 | rs12027476 | C/G | 0 | 25.9 | 25.9 | 4.391892 |
| FHR2 | rs12066959 | A/G | 5.5 | 25.3 | 30.8 | 1.013514 |
| FHR2 | rs4085749 | C/T | 30.5 | 25.1 | 5.4 | 0.337838 |
| FHR4 | rs1409153 | A/G | 25.6 | 6.9 | 32.5 | 0 |

**TABLE V**

| **Gene Name** | **Gene ID** |
|---|---|
| CFH | ENSG00000000971 |
| FHR1 | ENSG00000080910 |
| FHR2 | ENSG00000134391 |
| FHR3 | ENSG00000116785 |
| FHR4 | ENSG00000134365 |
| FHR5 | ENSG00000134389 |
| F13B | ENSG00000143278 |

**Table VI: Flanking Sequences for SNPs Associated with AMD**

| Gene | SNP | SNP Flanking Sequence |
|---|---|---|
| F13B | rs5997 | AAAATAAATAATTTTTATAATTTTAGAAACNTGTTTGGCTCCTGAATTATATAATGGAAAT |
| F13B | rs6428380 | agggaggcacaaaagtctggcttgcattctcNgctgggaggctagtagcctggggcaagttct |
| F13B | rs1794006 | aggggtagaggaagcaaagggtaaagccccNtcgtctctgtgggtccccagagaagccatt |
| F13B | rs10801586 | tagatctcatttgtcagttttggctctcatNgcaattgcttttggcattttcgtcattaag |
| FHR1 | rs12027476 | tatttgggcaggaatgtcccatttttcccagNtgcagtctgccatggcttcccttggctagga |
| FHR1 | rs436719 | tgccattaaatttttgactgactggccacttNgttgcttgccccagctaatatcctctacaca |
| FHR2 | rs12066959 | tcagaggatgtgaaaccAGTGGGGCTGACCNtatatatatgtgtgtatacaagtataaata |
| FHR2 | rs3828032 | gcaggtccactagtaagtgcaatgttgttctNtcagatgctgttatattataaagtgtaaaag |
| FHR2 | rs6674522 | ACAAGAAAAATTATTTTCTACTTTTGAAGTNGGTGGTTGTGTAAAGGAGGCTTGCAAGAAG |
| FHR2 | rs432366 | TGTTGAACCAATTTTACTTCAGAATAATTTTNTTCCGATGGGACTTTGAGAATGGGTATTTC |
| FHR4 | rs1409153 | tttaatatactattttgatcaaattcatgttNctaatctaccttttaatcattttatggtctt |
| FHR5 | MRD 3905 | tgcagaaaaggatgcgtgtgaacagcaggtaNttttcttctgattgattctatatctagatga |
| FHR5 | MRD_ 3906 | ggggaaaagcagtgtggaaattatttaggacNgtgttcattaatttaaagcaaggcaagtcag |
| FHR5 | rs10922153 | ataactttgaaactttctgaattaacgttatNtaaaaggaaatgtagatgttattttagtctc |

**Table VII: Flanking Sequences for SNPs Associated witht MPGNII**

| Gene | SNP | SNP Flanking Sequence |
|---|---|---|
| CFH | rs3753395 | ACAGGCCAATGACAAGTGTAACAAAAATGGNTTTTAATAGAGTAGAAGAGACAGACCCTAC |
| CFH | rs1410996 | CGTCCTGACTCAGTCCCTGACTACCTCATGNCACTCAGCTATACCACTGATGTAGAGGGCC |
| CFH | rs1329421 | tcaacattgttaaatttcatcttattagatNcagcttagcACATAAGAGTCTCTTTGAATG |
| CFH | rs10801554 | GAGGCATGAATTAACTATGTTATTTTTCTGNGCGGTATCATCAAAGAAAAATTTTTGTGTT |
| CFH | rs12124794 | TAATTGAGGCTAATAATATGCCTTGATTAGNTATGCAATTTCTCCTGATATCAAACAACTC |
| CFH | rs393955 | agccatcatacaaaagttatctctaaccaaNgtactcaaacagagtctttaccactgaaag |
| CFH | rs403846 | GTTTCTTTGCTTCTCAGTGCCTAAAAAGGANTACCATACAATAACaataatatttatattt |
| CFH | rs2284664 | ATATTAGAAAAATACCAGTCTCCATAGATCNTAAAGCAAATAGATGGTCTTAAAATGCTAT |
| CFH | rs12144939 | agattttctatttcctctgaattaatcgtcNtaggctgtgtgtctagaaatttatccattt |
| F13B | rs2990510 | GCCCTAAGTAGAGCAATGCTTTACAGTGTTNGTTGTTGAGTGCTCACAAGAAGGTGATCAA |
| FHR1 | rs12027476 | tatttgggcaggaatgtcccatttttcccagNtgcagtctgccatggcttcccttggctagga |
| FHR2 | rs12066959 | tcagaggatgtgaaaccAGTGGGGCTGACCNtatatatatgtgtgtatacaagtataaata |
| FHR2 | rs4085749 | tagaacggggctggtccactcctcccaaatgNaggtccactagtaagtgcaatgttgttctct |
| FHR4 | rs1409153 | tttaatatactattttgatcaaattcatgttNctaatctaccttttaatcattttatggtctt |

### RELEVANT PARAGRAPHS

1. A method of screening for susceptibility to complement dysregulation in an individual comprising screening for the presence or absence of a genetic profile characterized by polymorphisms in the genome of the individual associated with complement dysregulation, wherein the presence of the individual's risk of complement dysregulation, wherein the presence of a said genetic profile is indicative of the individual's risk of complement dysregulation, wherein the genetic profile comprises at least one polymorphism selected from Table I, Table IA or Table II.
2. A method of determining an individual's risk of development or progression age-related macular degeneration (AMD) comprising screening for the presence or absence of a genetic profile within the regulation of the complement activation (RCA) locus, wherein the genetic profile comprises one or more single nucleotide polymorphisms selected from Table I or Table IA.
3. A method of determining an individual's risk of development or progression of membranoproliferative glomerulonephritis type II (MPGNII) comprising screening for the presence or absence of a genetic profile in the regulation of the complement activation (RCA) locus, wherein the genetic profile comprises one or more single nucleotide polymorphisms selected from Table II.
4. The method of paragraph 1, 2 or 3 comprising screening for at least two of said polymorphisms.
5. The method of paragraph 1, 2 or 3 comprising screening for at least five of said polymorphisms.
6. The method of paragraph 1, 2 or 3 comprising screening for at least ten of said polymorphisms.
7. The method of paragraph 1, 2 or 3 comprising screening for a combination of at least one risk-associated polymorphism and at least one protective polymorphism.
8. The method of paragraph 1, 2 or 3 comprising screening for at least rs1409153, rs10922153, rs12066959, and rs12027476.
9. The method of paragraph 1, 2 or 3 comprising screening additionally for deletions within said region associated with AMD or MPGNII risk or protection.
10. The method of paragraph 1, 2 or 3 comprising screening for one or more additional AMD or MPGNII risk-associated or protection-associated polymorphisms in the genome of said individual.
11. The method of paragraph 10 comprising screening for an additional polymorphism selected from the group consisting of: a polymorphism in exon 22 of CFH (R1210C), rs2511989, rs1061170, rs203674, rs1061147, rs2274700, rs12097550, rs203674, rs9427661, rs9427662, rs10490924, rs11200638, rs2230199, rs800292, rs3766404, rs529825, rs641153, rs4151667, rs547154, rs9332739, rs2511989, rs3753395, rs1410996, rs393955, rs403846, rs1329421, rs10801554, rs12144939, rs12124794, rs2284664, rs16840422, and rs6695321.
12. The method of paragraph 1, 2 or 3 wherein the screening step is conducted by inspecting a data set indicative of genetic characteristics previously derived from analysis of the individual's genome.
13. The method of paragraph 1, 2 or 3 wherein the screening comprises analyzing a sample of said individual's DNA or RNA.
14. The method of paragraph 1, 2 or 3 wherein the screening comprises analyzing a sample of said individual's proteome to detect an isoform encoded by an allelic variant in a protein thereof consequent of the presence of a said polymorphism in said individual's genome.
15. The method of paragraph 1, 2 or 3 wherein the screening comprises (i) combining a nucleic acid sample from the subject with one or more polynucleotide probes capable of hybridizing selectively to DNA or RNA comprising a said polymorphism.
16. The method of paragraph 1, 2 or 3 wherein the screening comprises sequencing selected portions of the genome or transcriptome of said individual.
17. The method of paragraph 1, 2 or 3 wherein said individual is determined to be at risk of developing AMD or MPGNII symptoms comprising the additional step of prophylactically or therapeutically treating said individual to inhibit development of the symptoms.
18. The method of paragraph 1, 2 or 3 comprising the further step of producing a report identifying the individual and the identity of the alleles at the sites of said one or more polymorphisms.
19. A set of detectably labeled oligonucleotide probes for hybridization with at least two polymorphisms for identification of the base present in an individual's genome at the sites of said at least two polymorphisms, wherein the polymorphisms are selected from Table I, Table IA, or Table II.
20. A healthcare method comprising paying for, authorizing payment for or authorizing the practice of the method of paragraph 1, 2 or 3.
21. A method for treating or preventing AMD, the method comprising prophylactically or therapeutically treating an individual identified as having a genetic profile in the regulation of the complement activation (RCA) locus of chromosome one extending from FHR1 through F13B indicative of increased risk of development or progression of AMD, the genetic profile comprising one or more single nucleotide polymorphisms selected from Table I or Table IA.
22. A method for treating or preventing membranoproliferative glomerulonephritis type II (MPGNII), the method comprising prophylactically or therapeutically treating an individual identified as having a genetic profile in the regulation of the complement activation (RCA) locus of chromosome one extending from CFH through F13B indicative of increased risk of development or progression of MPGNII, the genetic profile comprising one or more single nucleotide polymorphisms selected from Table II.
23. The method of paragraph 21 or 22, comprising administering a factor H polypeptide to the individual.
24. The method of paragraph 23 wherein the factor H polypeptide is encoded by a factor H protective haplotype.
25. A healthcare method comprising paying for, authorizing payment for or authorizing the practice of the method of paragraph 21 or 22.
26. An oligonucleotide comprising a nucleotide sequence selected from the group consisting of SEQ ID NO: 3 and SEQ ID NO: 4 and their complements.
27. An oligonucleotide that hybridizes under stringent conditions to at least one of the SEQ ID NO: 1 and SEQ ID NO: 2 or its complement.
28. The oligonucleotide of paragraph 1, wherein the oligonucleotide is detectably labeled.

## Claims

1. An *in vitro* method of determining an individual's risk for age-related macular degeneration (AMD) comprising screening for the presence or absence of a genetic profile,
wherein the genetic profile comprises the individual being homozygous for the G allele at the polymorphic site rs1409153, and
wherein the presence of said genetic profile is indicative of increased risk.

2. The method of claim 1, wherein the genetic profile comprises at least one additional polymorphism indicative of increased risk, wherein the additional polymorphism is selected from: a G allele at the polymorphic site rs5997, a G allele at the polymorphic site rs6428380, a C allele at the polymorphic site rs1794006, a C allele at the polymorphic site rs10801586, a G allele at the polymorphic site rs12027476, an A allele at the polymorphic site rs436719, a G allele at the polymorphic site rs12066959, a G allele at the polymorphic site rs3828032, a G allele at the polymorphic site rs6674522, a G allele at the polymorphic site rs432366, a G allele at the polymorphic site rs10922153, a G allele at the polymorphic site MRD_3905, and a C allele at the polymorphic site MRD_3906.

3. The method of claim 2, wherein the genetic profile comprises an additional polymorphism selected from a G allele at the polymorphic site rs10922153; a G allele at the polymorphic site rs12066959; and, a G allele at the polymorphic site rs12027476.

4. The method of claim 1, 2, or 3, wherein the screening comprises analyzing a sample of said individual's DNA or RNA.

5. The method of claim 4, wherein the screening comprises combining a nucleic acid sample from the subject with one or more polynucleotide probes capable of hybridizing selectively to DNA or RNA comprising said allele or alleles.

6. The method of claim 1, 2, or 3, wherein the screening comprises sequencing selected portions of the genome or transcriptome of said individual.

7. The method of any one of claims 1 to 6, further comprising screening for deletions and/or screening for additional polymorphisms known to be associated with AMD risk or protection in the genome of said individual.

8. The method of claim 7, wherein the additional polymorphisms are selected from the group consisting of: rs1061170, rs203674, rs1061147, rs2274700, rs12097550, rs203674, rs9427661, rs9427662, rs10490924, rs11200638, rs2230199, rs800292, rs3766404, rs529825, rs641153, rs4151667, rs547154, rs9332739, rs2511989, rs3753395, rs1410996, rs393955, rs403846, rs1329421, rs10801554, rs12124794, rs2284664, rs16840422, rs6695321, or comprise a polymorphism in exon 22 of Complement Factor H (CFH) gene encoding cysteine rather than arginine at position 1210 of the CFH protein.

9. The method of any one of claims 1 to 8, comprising the further step of producing a report identifying the individual and the identity of alleles at polymorphic sites.

## Patentansprüche

1. Ein *In vitro*-Verfahren zur Bestimmung eines Risikos eines Individuums für altersbezogene Makuladegeneration (AMD), umfassend das Screening zum Vorhandensein oder Nichtvorhandensein eines genetischen Profils,
wobei das genetische Profil umfasst, dass das Individuum homozygot für das G-Allel an der polymorphen Stelle rs1409153 ist, und
wobei das Vorhandensein des besagten genetischen Profils ein Hinweis auf ein erhöhtes Risiko ist.

2. Das Verfahren nach Anspruch 1, wobei das genetische Profil mindestens einen zusätzlichen Polymorphismus umfasst, der indikativ für ein erhöhtes Risiko ist, wobei der zusätzliche Polymorphismus ausgewählt ist aus: einem G-Allel an der polymorphen Stelle rs5997, einem G-Allel an der polymorphen Stelle rs6428380, einem C-Allel an der polymorphen Stelle rs1794006, einem C-Allel an der polymorphen Stelle rs10801586, einem G-Allel an der polymorphen Stelle rs12027476, einem A-Allel an der polymorphen Stelle rs436719, einem G-Allel an der polymorphen Stelle rs12066959, einem G-Allel an der polymorphen Stelle rs3828032, einem G-Allel an der polymorphen Stelle rs6674522, einem G-Allel an der polymorphen Stelle rs432366, einem G-Allel an der polymorphen Stelle rs10922153, einem G-Allel an der polymorphen Stelle MRD_3905 und einem C-Allel an der polymorphen Stelle MRD_3906.

3. Das Verfahren nach Anspruch 2, wobei das genetische Profil einen zusätzlichen Polymorphismus umfasst ausgewählt aus einem G-Allel an der polymorphen Stelle rs10922153; einem G-Allel an der polymorphen Stelle rs12066959; und einem G-Allel an der polymorphen Stelle rs12027476.

4. Das Verfahren nach Anspruch 1, 2 oder 3, wobei das Screening das Analysieren einer Probe der DNA oder RNA des besagten Individuums umfasst.

5. Das Verfahren nach Anspruch 4, wobei das Screening das Kombinieren einer Nukleinsäure-Probe von dem Patienten mit einer oder mehreren Polynukleotid-Sonden umfasst, die selektiv zu DNA oder RNA hybridisieren können, die das Allel oder die Allele umfassen.

6. Das Verfahren nach Anspruch 1, 2 oder 3, wobei das Screening das Entschlüsseln ausgewählter Abschnitte des Genoms oder Transkriptoms des besagten Individuums umfasst.

7. Das Verfahren nach einem der Ansprüche 1 bis 6, ferner umfassend das Screening auf Deletionen und/oder Screening auf zusätzliche Polymorphismen, die bekanntermaßen mit einem AMD-Risiko oder-Schutz im Genom des besagten Individuums verbunden sind.

8. Das Verfahren nach Anspruch 7, wobei die zusätzlichen Polymorphismen ausgewählt sind aus der Gruppe bestehend aus: rs1061170, rs203674, rs1061147, rs2274700, rs12097550, rs203674, rs9427661, rs9427662, rs10490924, rs11200638, rs2230199, rs800292, rs3766404, rs529825, rs641153, rs4151667, rs547154, rs9332739, rs2511989, rs3753395, rs1410996, rs393955, rs403846, rs1329421, rs10801554, rs12124794, rs2284664, rs16840422, rs6695321 oder einen Polymorphismus in Exon 22 des Komplementfaktor H (CFH)-Gens umfassen, der Cystein und nicht Arginin an Position 1210 des CFH-Proteins codiert.

9. Das Verfahren nach einem der Ansprüche 1 bis 8, das den weiteren Schritt umfasst, einen Bericht zu fertigen, der das Individuum identifiziert und die Identität der Allele an den polymorphen Stellen kenntlich macht.

## Revendications

1. Procédé *in vitro* de détermination du risque de dégénérescence maculaire liée à l'âge (DMLA) d'un sujet, comprenant le dépistage de la présence ou de l'absence d'un profil génétique, dans lequel le profil génétique comprend le sujet étant homozygote pour l'allèle G au site polymorphe rs1409153 et dans lequel la présence dudit profil génétique indiquant un risque accru.

2. Procédé selon la revendication 1, dans lequel le profil génétique comprend au moins un polymorphisme supplémentaire indiquant un risque accru, dans lequel le polymorphisme supplémentaire est sélectionné parmi : un allèle G au site polymorphe rs5997, un allèle G au site polymorphe rs6428380, un allèle C au site polymorphe rs1794006, un allèle C au site polymorphe rs10801586, un allèle G au site polymorphe rs12027476, un allèle A au site polymorphe rs436719, un allèle G au site polymorphe rs12066959, un allèle G au site polymorphe rs3828032, un allèle G au site polymorphe rs6674522, un allèle G au site polymorphe rs432366, un allèle G au site polymorphe rs10922153 G, un allèle G au site polymorphe MRD_3905 et un allèle C au site polymorphe MRD_3906.

3. Procédé selon la revendication 2, dans lequel le profil génétique comprend un polymorphisme supplémentaire sélectionné parmi un allèle G au site polymorphe rs10922153 ; un allèle G au site polymorphe rs12066959 ; et, un allèle G au site polymorphe rs12027476.

4. Procédé selon la revendication 1, 2, ou 3, dans lequel le dépistage comprend l'analyse d'un échantillon d'ADN ou d'ARN du sujet.

5. Procédé selon la revendication 4, dans lequel le dépistage comprend la combinaison d'un échantillon d'acide nucléique provenant du sujet comportant une ou plusieurs sondes de polynucléotides susceptibles de s'hybrider de manière sélective à l'ADN ou l'ARN comprenant ledit allèle ou lesdits allèles.

6. Procédé selon la revendication 1, 2, ou 3, dans lequel le dépistage comprend le séquençage des portions sélectionnées du génome ou du transcriptome dudit sujet.

7. Procédé selon l'une quelconque des revendications 1 ou 6, comprenant en outre le dépistage destiné aux suppressions et/ou le dépistage destiné aux polymorphismes supplémentaires connus pour être associés avec le risque d'AMD ou la protection dans le génome dudit sujet.

8. Procédé selon la revendication 7, dans lequel les polymorphismes supplémentaires sont sélectionnés parmi le groupe constitué de : rs1061170, rs203674, rs1061147, rs2274700, rs12097550, rs203674, rs9427661, rs9427662, rs10490924, rs11200638, rs2230199, rs800292, rs3766404, rs529825, rs641153, rs4151667, rs547154, rs9332739, rs2511989, rs3753395, rs1410996, rs393955, rs403846, rs1329421, rs10801554, rs12124794, rs2284664, rs16840422, rs6695321, ou comprennent un polymorphisme dans exon 22 du gène du facteur H du complément (CFH) codant la cystéine plutôt que l'arginine à la position 1210 de la protéine CFH.

9. Procédé selon l'une quelconque des revendications 1 à 8, comprenant l'étape additionnelle de production d'un rapport identifiant le sujet et l'identité des allèles aux sites polymorphes.
